# EUROPEAN PATENT SPECIFICATION

(11) **EP 3 082 711 B1**
(45) Date of publication and mention of the grant of the patent: **08.09.2021**
(21) Application number: 13899763.0
(22) Date of filing: 18.12.2013
(51) Int. Cl.: A61K 8/02, A61K 8/25, A61K 8/26, A61K 8/34, A61K 8/37, A61K 8/89, A61K 8/92, A61Q 1/00, A61Q 5/00, A61Q 19/00, A61Q 1/02

(54) **COMPOSITION OF PICKERING EMULSION COMPRISING LOW AMOUNT OF ALCOHOL**
ZUSAMMENSETZUNG AUS EINER PICKERING-EMULSION MIT GERINGEN ALKOHOLMENGEN
COMPOSITION D'ÉMULSION DE PICKERING COMPRENANT UNE FAIBLE QUANTITÉ D'ALCOOL

(43) Date of publication of application: 26.10.2016
(73) Proprietor: L'Oréal, 75008 Paris (FR); Ma, Shouwei, Shanghai 200000 (CN)
(72) Inventor: MA, Shouwei, Shanghai 200000 (CN); LEMOINE, Cyril, 201206 Shanghai (CN)
(74) Representative: Casalonga
(86) International application number: PCT/CN2013/089751
(87) International publication number: WO 2015/089750

(56) References cited:
- EP-A2- 1 559 416
- WO-A1-2008/061985
- WO-A2-2007/070168
- US-A1- 2009 035 236
- US-A1- 2010 316 684
- None

## Description

### TECHNICAL FIELD

The present invention relates to the field of cosmetics, and especially to the field of compositions in the form of visible droplets in suspension in a liquid.

### BACKGROUND ART

Two-phase compositions which appeal to consumers on account of their aesthetic nature exist currently on the market. These compositions consist of two mutually immiscible phases, which are mixed together extemporaneously by shaking before use.

For example, EP 1 559 416 describes a cosmetic and/or dermatological oil-in-water (O/W) emulsion having a viscosity of from 100 to 1500 mPa.s. It comprises in particular glyceryl stearate citrate in combination with electrolytes and thickener in specific amounts.

Pickering emulsion, due to its aesthetic nature and surfactant-free property, is of great interest of the consumers and widely used in the cosmetic products. To form a Pickering emulsion, finely divided solid particles are adsorbed at the interface between the oil and the homogeneous mixture, and serve to stabilize the oil droplets.

However, these emulsions have a tendency to become destabilized on storage over time. The reason for this is that sedimentation of the solid particles or even phase separation is observed, leading to an appearance that consumers find unappealing.

FR1160798 disclosed a Pickering emulsion comprising apolar hydrocarbon-based oil, C₁-C₄ monoalcohol, and hydrophobic silica aerogel particles.

In order to stabilize the visible dispersed oily phase in the aqueous phase, a relatively high amount of alcohol is necessary. However, it may cause discomfort to the consumers. Moreover,

it is not a question in said document of Pickering emulsion comprising visible oil droplets dispersed in the aqueous phase.

There is thus a need for a composition of Pickering emulsion type with relatively low amount of monoalcohol, wherein the oil droplets are visible, and that has long-term stability.

### DISCLOSURE OF INVENTION

It is discovered that, in accordance to the present invention, a composition of Pickering emulsion type comprising a dispersed fatty phase, a continuous aqueous phase with low amount of alcohol, at least one hydrophobic particle, and at least one polysaccharide solves the problems mentioned above.

Thus, a subject of the present invention is a composition in form of an oil-in-water Pickering emulsion comprising:
a) a dispersed fatty phase comprising at least one oil chosen from silicone oils, hydrocarbon-based oils, or a mixture thereof;
b) a continuous aqueous phase comprising at least one C₁-C₄ alcohol, in an amount ranging from 1% to 10% by weight, relative to the total weight of the composition, the at least one C₁-C₄ alcohol comprising ethanol, ethylene glycol, C₁-C₄ dialkylene glycol or a mixture thereof;
c) 0.001% to 5% by weight, relative to the total weight of the composition, of at least one hydrophobic particle chosen from hydrophobic silicas, hydrophobic cellulose, starches, talc, silicone resin powders, hollow hemispherical silicone particles, polyamide powders, hydrophobic pigments, or a mixture thereof; and
d) at least one polysaccharide.

The other subject of the present invention is a process for making up/caring for the keratin materials, for example the skin, in particular the face and the lips, by applying to the keratin materials the composition of the present invention.

The term "Pickering emulsion" refers to an emulsion that is stabilized by solid particles (for example colloidal silica) which adsorb onto the interface between the two phases.

The term "keratin material" means the skin (of the body, face and around the eyes), hair, eyelashes, eyebrows, bodily hair, nails, lips or mucous membranes.

The term "visible oil droplets" of the present invention refers to the oil droplets with a median particle size by volume Dv50 ranging from 0.1 mm to 10 mm. The oil droplets are visible by observing them using the bear eyes.

The term "long term stability" means a composition that does not undergo any significant change in its structure or properties for at least one month after its manufacture and especially for at least two months after its manufacture.

### DETAILED DESCRIPTION OF THE INVENTION

The composition according to the present invention relates to a composition in form of an oil-in-water Pickering emulsion comprising:
a) a dispersed fatty phase comprising at least one oil chosen from silicone oils, hydrocarbon-based oils, or a mixture thereof;
b) a continuous aqueous phase comprising at least one C₁-C₄ alcohol, in an amount ranging from 1% to 10% by weight, relative to the total weight of the composition, the at least one C₁-C₄ alcohol comprising ethanol, ethylene glycol, C₁-C₄ dialkylene glycol or a mixture thereof;
c) 0.001% to 5% by weight, relative to the total weight of the composition, of at least one hydrophobic particle chosen from hydrophobic silicas, hydrophobic cellulose, starch, talc, silicone resin powders, hollow hemispherical silicone particles, polyamides powders, hydrophobic pigments, or a mixture thereof; and
d) at least one polysaccharide.

### Fatty phase

According to the present invention, the composition comprises a dispersed fatty phase.

In particular, the fatty phase of the present invention is in form of droplets.

More particularly, the droplets have a median particle size by volume Dv50 from 0.1 mm to 10 mm, preferably from 0.5 mm to 5 mm.

The median particle size by volume Dv50 is a parameter for particle size distribution, referring to the maximum particle diameter below which 50% of the sample volume exists (see in A Basic Guide To Particle Characterization, page 10, published by Malvern Instruments Limited in 2012).

The particle size by volume Dv50 of the oil droplets may be measured by static light scattering using a commercial granulometer such as the MasterSizer 3000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C., "Light Scattering by Small Particles," Chapters 9 and 10, Wiley, New York, 1957.

Preferably, the composition may comprise a fatty phase presenting in the composition in a content ranging from 0.1% to 40% by weight, preferably from 1% to 30% by weight, and more preferably from 3% to 20% by weight relative to the total weight of the composition.

### Oils

A composition in accordance with the present invention comprises a dispersed fatty phase, wherein it comprises at least one oil chosen from silicone oils, hydrocarbon-based oils, or a mixture thereof.

The oil can be volatile or non-volatile.

The term "volatile" means an oil that is capable of evaporating on contact with keratin materials in less than one hour, at room temperature (25°C) and atmospheric pressure (760 mmHg). The volatile oil is a volatile cosmetic oil, which is liquid at room temperature, especially having a non-zero vapour pressure, at room temperature and atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg) and preferentially ranging from 1.3 Pa to 1300 Pa (0.1 to 10 mmHg).

The term "non volatile" means an oil whose vapour pressure at room temperature and atmospheric pressure is non-zero and less than 0.02 mmHg (2.66 Pa) and better still less than 10⁻³ mmHg (0.13 Pa).

### 1. Silicone oils

The term "silicone oil" is intended to mean an oil comprising at least one silicon atom, and in particular comprising Si-O group.

The non volatile silicone oil that may be used in the invention may be chosen especially from silicone oils especially with a viscosity at 25°C of greater than or equal to 9 centistokes (cSt) (9 x 10⁻⁶ m²/s) and preferably less than 800 000 cSt, preferably between 50 and 600 000 cSt and preferably between 100 and 500 000 cSt. The viscosity of this silicone oil may be measured according to standard ASTM D-445.

Among these silicone oils, two types of oil may be distinguished, according to whether or not they contain phenyl.

### A. Non volatile Phenyl silicone oil or non volatile phenylated silicone oil

A composition according to the invention contains at least one non volatile phenyl silicone oil.

The expression "phenylated silicone oil" or "phenyl silicone oil" means a silicone oil having at least one phenyl substituent.

The phenyl silicone oil can be chosen among those having at least a dimethicone, or those having no dimethicone part.

According to the present invention, the dimethicone part corresponds to the formula: -Si(CH₃)-O-.

More particularly, the non volatile phenyl silicone oil may be chosen from:
a) the phenyl silicone oils corresponding to the following formula (I), with or without a dimethicone part: 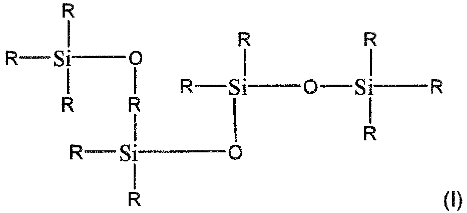 in which the groups R represent, monovalent or divalent, independently of each other, a methyl or a phenyl, with the proviso that at least one group R represents a phenyl.
   Preferably, in this formula, the phenyl silicone oil comprises at least three phenyl groups, for example at least four, at least five or at least six.
b) the phenyl silicone oils corresponding to the following formula (II), with or without a dimethicone part: 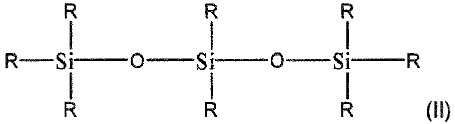 in which the groups R represent, independently of each other, a methyl or a phenyl, with the proviso that at least one group R represents a phenyl.
   Preferably, in this formula, the phenyl silicone oil comprises at least three phenyl groups, for example at least four or at least five.
   Mixtures of the phenyl organopolysiloxanes described previously may be used.
   Examples that may be mentioned include mixtures of triphenyl-, tetraphenyl- or pentaphenyl-organopolysiloxanes.
   Among the compounds of formula (II), a preferred embodiment is made in particular to a phenyl silicone oil without a dimethicone part and correspond to the formula (II) wherein at least 4 or at least 5 of the group R represent phenyl, and the remaining R groups represent methyl.
   Such phenyl silicone oils are preferably trimethyl pentaphenyl trisiloxane, or tetramethyl tetraphenyl trisiloxane. Such oils are especially manufactured by Dow Corning under the reference PH-1555 HRI or Dow Corning 555 Cosmetic Fluid (chemical name: 1,3,5-trimethyl-1,1,3,5,5-pentaphenyl trisiloxane; INCI name: trimethyl pentaphenyl trisiloxane), or tetramethyl tetraphenyl trisiloxane sold under the reference Dow Corning 554 Cosmetic Fluid by Dow Corning may also be used.
   In particular, these silicone oils correspond to the formulas (III), or (III'): 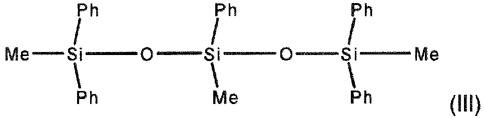 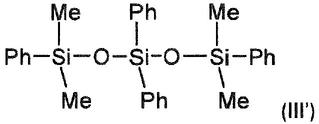 in which Me represents methyl, Ph represents phenyl.
c) the phenyl silicone oils corresponding to the following formula (IV), with or without a dimethicone part: 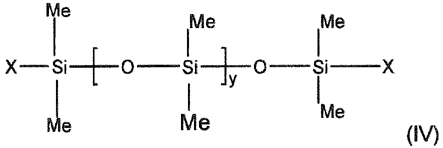 in which Me represents methyl, y is between 1 and 1,000 and X represents -CH₂-CH(CH₃)(Ph).
d) the phenyl silicone oils corresponding to formula (V) below, and the mixtures thereof: 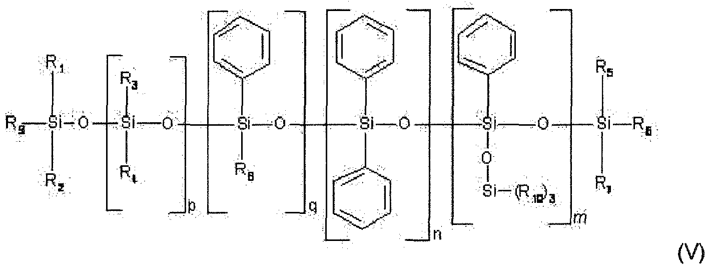 in which:
   - R₁ to R₁₀, independently of each other, are saturated or unsaturated, linear, cyclic or branched C₁-C₃₀ hydrocarbon-based radicals,
   - m, n, p and q are, independently of each other, integers between 0 and 900, with the proviso that the sum m+n+q is other than 0.

Preferably, the sum m+n+q is between 1 and 100. Preferably, the sum m+n+p+q is between 1 and 900 and better still between 1 and 800. Preferably, q is equal to 0.

Preferably, R₁ to R₁₀, independently of each other, represent a saturated or unsaturated linear or branched C₁-C₃₀, preferably C₁-C₂₀, in particular C₁-C₁₆, hydrocarbon radical, preferably saturated, or a monocyclic or polycyclic C₆-C₁₄ and especially C₁₀-C₁₃ aryl radical, or an aralkyl radical.

Preferably, R₁ to R₁₀ may each represent a methyl, ethyl, propyl, butyl, isopropyl, decyl, dodecyl or octadecyl radical, or alternatively a phenyl, tolyl, benzyl or phenethyl radical. R₁ to R₁₀ may especially be identical, and in addition may be a methyl radical.

According to a preferred embodiment, the present invention of the formula (V) can be:
i) the phenyl silicones oils corresponding to the formula (VI) below, and mixtures thereof: 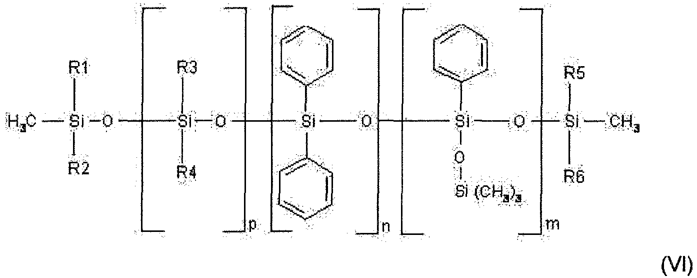 in which:
   - R₁ to R₆, independently of each other, are saturated or unsaturated, linear, cyclic or branched C₁-C₃₀ hydrocarbon-based radicals, preferably R₁ to R₆ are a C₁-C₃₀ alkyl radical, an aryl radical or an aralkyl radical, more preferably a C₆-C₁₄ aryl radical, or a C₁-C₃ alkyl radical or aralkyl radical;
   - m, n and p are, independently of each other, integers between 0 and 100, with the proviso that the sum n+m is between 1 and 100.

   Preferably, R₁ to R₆, independently of each other, represent a saturated or unsaturated linear or branched C₁-C₃₀, hydrocarbon radical, preferably saturated, and especially C₁-C₂₀, or C₁-C₁₆ hydrocarbon-based radical, in particular C₃-C₁₆ and more particularly C₄-C₁₀, or a monocyclic or polycyclic C₆-C₁₄ and especially C₁₀-C₁₃ aryl radical, or an aralkyl radical. Preferably, R₁ to R₆ may each represent a methyl, ethyl, propyl, butyl, isopropyl, decyl, dodecyl or octadecyl radical, or alternatively a phenyl, tolyl, benzyl or phenethyl radical.
   R₁ to R₆ may especially be identical, and in addition may be a methyl radical. Preferably, m = 1 or 2 or 3, and/or n = 0 and/or p = 0 or 1 may apply, in formula (VI).
   According to a particular embodiment, the non volatile phenyl silicone oil of the present invention is chosen from the phenyl silicone oils having at least a dimethicone part.
   Preferably the said oil corresponds to the formula (VI), wherein:
   A) m = 0 and n and p, independently of each other, are integers between 1 and 100. Preferably R₁ to R₆ are methyl group.
      Examples of such silicone oils are, for example, phenyl dimethicones, phenyltrimethylsiloxydiphenylsiloxanes, diphenyl dimethicones (such as KF-54 from Shin Etsu (400 cSt), KF54HV from Shin Etsu (5000 cSt), KF-50-300CS from Shin Etsu (300 cSt), KF-53 from Shin Etsu (175cSt), KF-50-100CS from Shin Etsu (100 cSt).
   B) p is an integer between 1 and 100, the sum of n + m is between 1 and 100, and more preferably n = 0.

   Such phenyl silicone oils with or without at least one dimethicone part correspond in particular to the formula (VII) 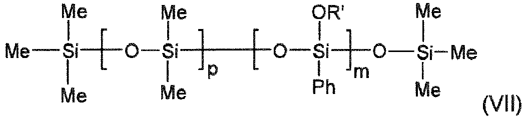
   in which Me is methyl and Ph is phenyl, OR' represents a group -OSiMe₃ and p is 0 or ranges between 1 and 1000, and m ranges between 1 and 1000, in particular, m and p are such that compound (VII) is a non-volatile oil.
   According to a first embodiment of the non volatile phenyl silicone oil having at least one dimethicone part, p ranges between 1 and 1000, m is in particular such that the compound (VII) is a non-volatile oil. Use may be made, for example, of trimethyl siloxyphenyl dimethicone, sold especially under the reference Belsil PDM 1000 sold by the company Wacker.
   According to a second embodiment of the non volatile phenyl silicone oil without a dimethicone part, p is equal to 0, m ranges between 1 and 1000, and in particular, is such that the compound (VII) is a non-volatile oil. Use may be made, for example, of phenyl trimethylsiloxy trisiloxane, sold especially under the reference Dow Corning 556 Cosmetic Grade Fluid (DC556).
ii) the phenyl silicone oils without a dimethicone part, corresponding to the formula (VIII) below, and mixtures thereof: 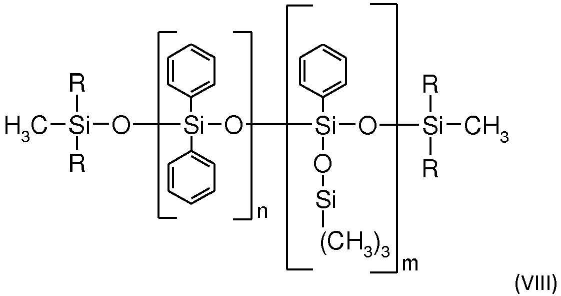 in which:
   - R, independently of each other, are saturated or unsaturated, linear, cyclic or branched C₁-C₃₀ hydrocarbon-based radicals, preferably R is a C₁-C₃₀ alkyl radical, an aryl radical or an aralkyl radical, more preferably R is a C₆-C₁₄ aryl radical, or a aralkyl radical with the C₁-C₃ alkyl group.
   - m and n are, independently of each other, integers between 0 and 100, with the proviso that the sum n+m is between 1 and 100.

Preferably, R, independently of each other, represent a saturated or unsaturated linear or branched C₁-C₃₀ hydrocarbon-based radical, preferably saturated, and especially C₁-C₁₂ hydrocarbon-based radical, in particular C₃-C₁₆ and more particularly C₄-C₁₀, or a monocyclic or polycyclic C₆-C₁₄ and especially C₁₀-C₁₃ aryl radical, or an aralkyl radical.

Preferably, R may each represent a methyl, ethyl, propyl, butyl, isopropyl, decyl, dodecyl or octadecyl radical, or alternatively a phenyl, tolyl, benzyl or phenethyl radical.

R may especially be identical, and in addition may be a methyl radical.

Preferably, m = 1 or 2 or 3, and/or n = 0 and/or p = 0 or 1 may apply, in formula (VIII). According to a preferred embodiment, n is an integer between 0 and 100 and m is an integer between 1 and 100, with the proviso that the sum n+m is between 1 and 100, in formula (VIII). Preferably R is methyl radical.

According to a preferred embodiment, n is an integer ranging between 0 and 100, and m is an integer ranging between 1 and 100, with the proviso that the sum n+m ranges between 1 and 100, in the formula (VIII). More preferably R is a methyl radical.

According to one embodiment, a phenyl silicone oil of formula (VIII) with a viscosity at 25°C of between 5 and 1500 mm2/s (i.e. 5 to 1500 cSt), and preferably with a viscosity of between 5 and 1000 mm2/s (i.e. 5 to 1000 cSt) may be used.

According to this embodiment, the non volatile phenyl silicone oil is preferably chosen from phenyl trimethicones; such as DC556 from Dow Corning (22.5 cst, the oil diphenylsiloxy phenyltrimethicone such as KF-56 A from Shin Etsu, the oil Silbione 70663V30 from Rhône-Poulenc (28 cSt). The values in parentheses represent the viscosities at 25°C.

According to this embodiment, when n=0, said silicone oil is preferably DC556 from Dow Corning, and when m and n are between 1 and 100, said silicone oil is preferably KF-56 A from Shin Etsu.
e) the phenyl silicone oils corresponding to the following formula (IX), with or without a dimethicone part, and mixtures thereof: 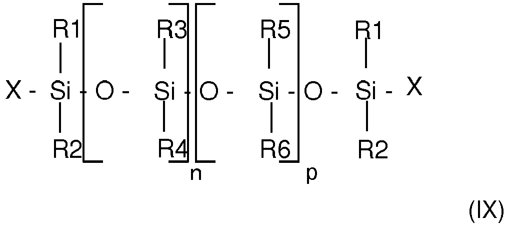 in which:
   R₁, R₂, R₅ and R₆ are, together or separately, an alkyl radical containing 1 to 6 carbon atoms, R₃ and R₄ are, together or separately, an alkyl radical containing from 1 to 6 carbon atoms or an aryl radical, with the proviso that at least one from R₃ and R₄ is a phenyl radical,
   X is an alkyl radical containing from 1 to 6 carbon atoms, a hydroxyl radical or a vinyl radical, n and p being integer superior or equal to 1, chosen so as to give the oil a weight-average molecular mass of less than 200 000 g/mol, preferably less than 150 000 g/mol and more preferably less than 100 000 g/mol.
f) and mixture thereof.
   As preferred non-volatile silicone oils, with or without a dimethicone part, examples that may be mentioned include silicone oils such as:
   - non volatile phenyl silicone oil, preferably chosen from : Tetramethyl Tetraphenyl Trisiloxane (such as as PH-1554 HRI or Dow Corning 554 Cosmetic Fluid from Dow Corning), trimethylsiloxyphenyldimethicone (for instance Belsil PDM 1000 from the company Wacker (cf. formula (V) above)), phenyl trimethicones (such as the phenyl trimethicone sold under the trade name DC556 by Dow Corning), phenyl dimethicones, phenyltrimethylsiloxydiphenylsiloxanes, diphenyl dimethicones (such as KF-54 from Shin Etsu (400 cSt), KF54HV from Shin Etsu (5000 cSt), KF-50-300CS from Shin Etsu (300 cSt), KF-53 from Shin Etsu (175cSt), KF-50-100CS from Shin Etsu (100 cSt), diphenylmethyldiphenyltrisiloxanes, 2-phenylethyl trimethylsiloxysilicates, trimethylpentaphenyl trisiloxane (such as the product sold under the name Dow Corning PH-1555 HRI Cosmetic fluid by Dow Corning) (cf. formula (III) above), diphenylsiloxy phenyltrimethicone (such as KF-56 A from Shin Etsu),
   - non volatile polydimethylsiloxanes (PDMS), polydimethylsiloxanes comprising alkyl or alkoxy groups, which are pendent and/or at the end of the silicone chain, these groups each containing from 2 to 24 carbon atoms, and
   - mixtures thereof.

According to a preferred embodiment, the composition of the present invention comprises, in a dispersed fatty phase, at least one non volatile phenyl silicone oil.

The non volatile phenyl silicone oils are more preferably chosen from:
- phenyl trimethicones,
- diphenylsiloxyphenyltrimethicone,
- trimethylpentaphenyl trisiloxane,
- trimethylsiloxyphenyldimethicone, and
- mixtures thereof.

### B. Non volatile non phenyl silicone oil

The composition according to the invention may also comprise a non volatile non phenyl silicone oil.

The expression "non volatile non phenyl silicone oil" or "non phenylated non volatile silicone oil" are equivalent and both means a silicon oil having no phenyl substituent.

Representative examples of these non volatile non phenyl silicone oils that may be mentioned include polydimethylsiloxanes; alkyl dimethicones; vinyl methyl methicones; and also silicones modified with optionally fluorinated aliphatic groups, or with functional groups such as hydroxyl, thiol and/or amine groups.

The non volatile non phenyl silicon oil is preferably chosen from dimethicone oils, preferably chosen from polydimethylsiloxanes; alkyl dimethicones.

"Dimethicone" (INCI Name) corresponds to polydimethylsiloxane (chemical name).

Non volatile non phenyl silicone oils can be chosen from:
- non volatile polydimethylsiloxanes (PDMS),
- PDMSs comprising alkyl or alkoxy groups, which are pendent and/or at the end of the silicone chain, these groups each containing from 2 to 24 carbon atoms, such as cetyldimethicone sold under the commercial reference ABIL WAX 9801 from Evonik Goldschmidt,
- PDMSs comprising aliphatic and/or aromatic groups, or functional groups such as hydroxyl, thiol and/or amine groups,
- polyalkylmethylsiloxanes such as cetyldimethicone sold under the commercial reference ABIL WAX 9801 from Evonik Goldschmidt, or polyalkylmethylsiloxane optionally substituted with a fluorinated group, such as polymethyltrifluoropropyldimethylsiloxanes,
- polyalkylmethylsiloxanes substituted with functional groups such as hydroxyl, thiol and/or amine groups,
- polysiloxanes modified with fatty acids, fatty alcohols or polyoxyalkylenes, and mixtures thereof.

These non volatile non phenyl linear silicone oils may be chosen from polydimethylsiloxanes; alkyl dimethicones; vinyl methyl methicones; and also silicones modified with optionally fluorinated aliphatic groups, or with functional groups such as hydroxyl, thiol and/or amine groups.

The non volatile non phenyl linear silicone oil may be chosen especially from the silicones of formula (I'): 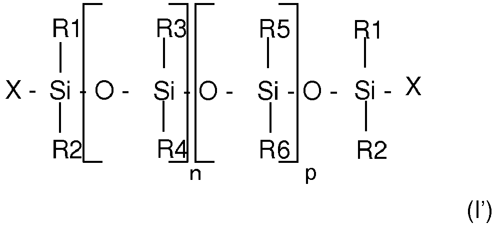 in which:
R₁, R₂, R₅ and R₆ are, together or separately, an alkyl radical containing 1 to 6 carbon atoms,
R₃ and R₄ are, together or separately, an alkyl radical containing from 1 to 6 carbon atoms, a vinyl radical, an amine radical or a hydroxyl radical,
X is an alkyl radical containing from 1 to 6 carbon atoms, a hydroxyl radical or an amine radical, n and p are integers chosen so as to have a fluid compound, in particular whose viscosity at 25°C is between 9 centistokes (cSt) (9 x 10-6 m2/s) and 800 000 cSt.

As non-volatile non phenylated silicone oils that may be used according to the invention, mention may be made of those for which:
- the substituents R₁ to R₆ and X represent a methyl group, and p and n are such that the viscosity is 500 000 cSt, such as the product sold under the name SE30 by the company General Electric, the product sold under the name AK 500000 by the company Wacker, the product sold under the name Mirasil DM 500 000 by the company Bluestar, and the product sold under the name Dow Corning 200 Fluid 500 000 cSt by the company Dow Corning,
- the substituents R₁ to R₆ and X represent a methyl group, and p and n are such that the viscosity is 60 000 cSt, such as the product sold under the name Dow Corning 200 Fluid 60000 CS by the company Dow Corning, and the product sold under the name Wacker Belsil DM 60 000 by the company Wacker,
- the substituents R₁ to R₆ and X represent a methyl group, and p and n are such that the viscosity is 350 cSt, such as the product sold under the name Dow Corning 200 Fluid 350 CS by the company Dow Corning,
- the substituents R₁ to R₆ represent a methyl group, the group X represents a hydroxyl group, and n and p are such that the viscosity is 700 cSt, such as the product sold under the name Baysilone Fluid T0.7 by the company Momentive.

For the purposes of the invention, the composition may comprise volatile silicone oil.

The volatile silicone oil that may be used in the invention may be chosen from silicone oils especially having a viscosity ≤ 8 centistokes (cSt) (8 x 10-6 m2/s) and preferably greater than 0.5 cSt.

The volatile silicone oil that can be used in the invention may be chosen from silicone oils having a flash point ranging from 40°C to 150°C, preferably having a flash point of greater than 55°C and less than or equal to 105°C, and preferentially ranging from 65°C to 95°C. The flash point is in particular measured according to ISO standard 3679.

The volatile silicone oil may be chosen from linear or cyclic silicone oils such as linear or cyclic polydimethylsiloxanes (PDMSs) having from 3 to 7 silicon atoms.

Volatile silicone oils that may more particularly be mentioned include decamethylcyclopentasiloxane sold especially under the name DC-245 by the company Dow Corning, dodecamethylcyclohexasiloxane sold especially under the name DC-246 by the company Dow Corning, octamethyltrisiloxane sold especially under the name DC-200 Fluid 1 cSt by the company Dow Corning, polydimethylsiloxanes such as decamethyltetrasiloxane sold especially under the name DC-200 Fluid 1.5 cSt by the company Dow Corning and DC-200 Fluid 5 cSt sold by the company Dow Corning, octamethylcyclotetrasiloxane, heptamethylhexyltrisiloxane, heptamethylethyltrisiloxane, heptamethyloctyltrisiloxane and dodecamethylpentasiloxane, octyl trimethicone, hexyl trimethicone, decamethylcyclopentasiloxane (cyclopentasiloxane or D5), octamethylcyclotetrasiloxane (cyclotetradimethylsiloxane or D4), dodecamethylcyclohexasiloxane (D6), decamethyltetrasiloxane (L4), KF 96 A from Shin Etsu, and mixtures thereof.

### 2. Hydrocarbonated oil

The term "hydrocarbon-based oil" (or "hydrocarbonated oil", or "hydrocarbon oil") means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

These oils may be of plant, mineral or synthetic origin.

### A. Non volatile hydrocarbonated apolar oil

The composition according to the invention may comprise at least one non volatile apolar hydrocarbonated oil (also called apolar "hydrocarbon-based" oil).

For the purposes of the present invention, the term "apolar oil" means an oil whose solubility parameter at 25°C, 8a, is equal to 0 (J/cm3)1/2.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:
- δD characterizes the London dispersion forces derived from the formation of dipoles induced during molecular impacts;
- δp characterizes the Debye interaction forces between permanent dipoles and also the Keesom interaction forces between induced dipoles and permanent dipoles;
- δh characterizes the specific interaction forces (such as hydrogen bonding, acid/base, donor/acceptor, etc.); and
- 8a is determined by the equation: 8a = (δp2 + δh2)½.

The parameters δp, δh, δD and δa are expressed in (J/cm3)½.

Preferably, the non volatile hydrocarbonnated apolar oil may be chosen from linear or branched hydrocarbons of mineral or synthetic origin.

In particular said non volatile hydrocarbonated apolar oil may be chosen from:
- liquid paraffin or derivatives thereof,
- squalane,
- isoeicosane,
- naphthalene oil,
- polybutylenes such as Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes,
- hydrogenated polyisobutylenes such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers, polybutene/polyisobutene copolymers, especially Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical), and
- mixtures thereof.

Preferably, the composition according to the invention comprises at least one non volatile hydrocarbon-based apolar oil, preferably chosen from polybutenes, polyisobutenes, hydrogenated polyisobutenes, polydecenes and/or hydrogenated polydecenes, and mixtures thereof.

### B. Non volatile hydrocarbonated polar oil

According to a preferred embodiment, the composition according to the invention may comprise one or more non volatile polar hydrocarbonated oil.

For the purposes of the present invention, the term "polar oil" means an oil whose solubility parameter at 25°C, 8a, is other than 0 (J/cm3)1/2.

These oils may be of plant, mineral or synthetic origin.

In particular, the additional non volatile hydrocarbonated polar oil may be chosen from the list of oils below, and mixtures thereof:
- hydrocarbonated plant oils such as liquid triglycerides of fatty acids containing from 4 to 10 carbon atoms, for instance heptanoic or octanoic acid triglycerides, jojoba oil, or caprylic and/or capric acid triglycerides, for example the one sold under the tradename Myritol® 318 by the company Cognis (BASF);
- ester oils, preferably chosen from:
- fatty acid esters, in particular of 4 to 22 carbon atoms, and especially of octanoic acid, heptanoic acid, lanolic acid, oleic acid, lauric acid or stearic acid, for instance propylene glycol dioctanoate, propylene glycol monoisostearate or neopentyl glycol diheptanoate;
- synthetic esters, for instance the oils of formula R₁COOR₂ in which R₁ represents a linear or branched fatty acid residue comprising from 4 to 40 carbon atoms and R₂ represents a hydrocarbon-based chain, which is especially branched, containing from 4 to 40 carbon atoms, on condition that R₁ + R₂ ≥ 16, for instance purcellin oil (cetostearyl octanoate), isononyl isononanoate, C₁₂ to C₁₅ alkyl benzoate, 2-ethylhexyl palmitate, octyldodecyl neopentanoate, 2-octyldodecyl stearate, 2-octyldodecyl erucate, oleyl erucate, isostearyl isostearate, 2-octyldodecyl benzoate, alcohol or polyalcohol octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate or 2-diethylhexyl succinate; preferably, the preferred synthetic esters R₁COOR₂ in which R₁ represents a linear or branched fatty acid residue comprising from 4 to 40 carbon atoms and R₂ represents a hydrocarbon-based chain, which is especially branched, containing from 4 to 40 carbon atoms are such that R₁ and R₂ ≥ 20;
- linear fatty acid esters with a total carbon number ranging from 35 to 70, for instance pentaerythrityl tetrapelargonate (MW = 697 g/mol);
- hydroxylated esters, preferably with a total carbon number ranging from 35 to 70, for instance polyglyceryl-2 triisostearate (MW = 965 g/mol), isostearyl lactate, octyl hydroxystearate, octyldodecyl hydroxystearate, diisostearyl malate, glyceryl stearate; diethylene glycol diisononanoate;
- esters of aromatic acids and of alcohols comprising 4 to 22 atoms, such as tridecyl trimellitate (MW = 757 g/mol);
- C₂₄-C₂₈ esters of branched fatty alcohols or fatty acids such as those described in patent application EP-A-0 955 039, and especially triisoarachidyl citrate (MW = 1033.76 g/mol), pentaerythrityl tetraisononanoate (MW = 697 g/mol), glyceryl triisostearate (MM = 891 g/mol), glyceryl tris(2-decyl)tetradecanoate (MW = 1143 g/mol), pentaerythrityl tetraisostearate (MW = 1202 g/mol), polyglyceryl-2 tetraisostearate (MW = 1232 g/mol) or pentaerythrityl tetrakis(2-decyl)tetradecanoate (MW = 1538 g/mol),
- polyesters resulting from the esterification of at least one hydroxylated carboxylic acid triglyceride with an aliphatic monocarboxylic acid and with an aliphatic dicarboxylic acid, which is optionally unsaturated, for instance the succinic acid and isostearic acid castor oil sold under the reference Zenigloss by Zenitech;
- esters of a diol dimer and of a diacid dimer of general formula HO-R₁-(-OCO-R₂-COO-R₁-)ₕ-OH, in which:
   R₁ represents a diol dimer residue obtained by hydrogenation of dilinoleic diacid,
   R₂ represents a hydrogenated dilinoleic diacid residue, and
   h represents an integer ranging from 1 to 9,
   especially the esters of dilinoleic diacids and of dilinoleyl diol dimers sold by the company Nippon Fine Chemical under the trade names Lusplan DD-DA5® and DD-DA7®,
- polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or copolymers of polyols and of diacid dimers, and esters thereof, such as Hailuscent ISDA;
- fatty alcohols containing from 12 to 26 carbon atoms, which are preferably branched, for instance octyldodecanol, 2-butyloctanol, 2-hexyldecanol, 2-undecylpentadecanol and oleyl alcohol;
- fatty acids containing from 12 to 26 carbon atoms, for instance oleic acid;
- oils of plant origin, such as sesame oil (820.6 g/mol);
- dialkyl carbonates, the two alkyl chains possibly being identical or different, such as dicaprylyl carbonate sold under the name Cetiol CC® by Cognis; and
- vinylpyrrolidone copolymers such as the vinylpyrrolidone/1-hexadecene copolymer, Antaron V-216 sold or manufactured by the company ISP (MW = 7300 g/mol).

Preferably, the composition according to the invention comprises at least one additional non volatile polar hydrocarbon oil chosen from oils from plant origin preferably chosen from liquid triglycerides of fatty acids, even more preferably heptanoic/octanoic triglycerides, caprylic/capric triglycerides, jojoba oils, or a mixture thereof.

### C. Volatile hydrocarbonated oil

According to a preferred embodiment, the composition according to the invention further comprises a volatile hydrocarbonated oil such as isododecane and/or isohexadecane.

Such compound is compatible with the non volatile hydrocarbonated and silicone oil and improves the spreadability during application and the transfer resistance of the deposit.

The volatile hydrocarbon-based oils may be chosen from hydrocarbon-based oils containing from 8 to 16 carbon atoms, and especially C₈-C₁₆ branched alkanes (also known as isoparaffins), for instance isododecane (also called 2,2,4,4,6-pentamethylheptane), isodecane and isohexadecane, and mixture thereof.

The volatile hydrocarbon-based oil may also be a linear volatile alkane containing 7 to 17 carbon atoms, in particular 9 to 15 carbon atoms and more particularly 11 to 13 carbon atoms. Mention may be made especially of n-nonadecane, n-decane, n-undecane, n-dodecane, n-tridecane, n-tetradecane, n-pentadecane and n-hexadecane, and mixtures thereof.

As other volatile hydrocarbon-based solvents (oils) that can be used in the composition according to the invention, mention may also be made of ketones which are liquid at ambient temperature, such as methyl ethyl ketone or acetone; short-chain esters (containing from 3 to 8 carbon atoms in total), such as ethyl acetate, methyl acetate, propyl acetate or n-butyl acetate; ethers which are liquid at ambient temperature, such as diethyl ether, dimethyl ether or dichlorodiethyl ether; alcohols, and in particular linear or branched lower monoalcohols containing from 2 to 5 carbon atoms, for instance ethanol, isopropanol or n-propanol.

According to an embodiment, the composition of the present invention comprises from 0.1% to 40% by weight, preferably from 1% to 30% by weight, and more preferably from 3% to 20% by weight of at least one oil, relative to the total weight of the composition.

According to another embodiment, the composition of the present invention comprises from 0.1 % to 40% by weight, preferably from 1% to 30% by weight, and more preferably from 3% to 20% by weight of at least silicone oil, relative to the total weight of the composition.

According to yet another embodiment, the composition of the present invention comprises from 0.1% to 40% by weight, preferably from 1% to 30% by weight, and more preferably from 3% to 20% by weight of at least non volatile phenyl silicone oil, relative to the total weight of the composition.

### Aqueous phase

The composition according to the invention comprises a continuous aqueous phase.

The continuous aqueous phase comprises at least one C₁-C₄ alcohol, in an amount of 1% to 10% by weight, relative to the total weight of the composition.

A alcohol according to the present invention comprises ethanol, diethylene glycol, C₁-C₄ dialkylene alcohol, or a mixture thereof.

Preferably the alcohol of the present invention is chosen from ethanols, ethylene glycol, or a mixture thereof.

According to an even preferred embodiment, the C₁-C₄ alcohol of the present invention is ethanol.

The continuous aqueous phase comprises water.

The continuous aqueous phase may also comprise water-miscible organic solvents (at room temperature of 20-25°C), for instance polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, or dipropylene glycol; glycol ethers (especially containing from 3 to 16 carbon atoms) such as mono-, di- or tripropylene glycol (C₁-C₄)alkyl ethers, mono-, di- or triethylene glycol (C₁-C₄)alkyl ethers, and mixtures thereof.

The aqueous phase may also comprise stabilizers, for example sodium chloride, magnesium dichloride or magnesium sulfate.

In particular, a composition of the invention may comprise an aqueous phase in an amount ranging from 55% to 99.9% by weight, especially from 60% to 90% and more particularly from 65% to 85% by weight relative to the total weight of the composition.

### Hydrophobic particles

A composition according to the present invention comprises at least one hydrophobic particle chosen from hydrophobic silicas, hydrophobic cellulose, starch, talc, silicone resin powders, hollow hemispherical silicone particles, polyamide powders, hydrophobic pigments, or a mixture thereof.

For the purpose of the invention, these particles adsorb onto the interface between the fatty phase and aqueous phase, so as to stabilize the emulsion.

Moreover, these particles enables the oil droplets of the present invention, which are visible, to be dispersed into the aqueous phase in a long-term, for example for one month, or for example for two months.

The paritcles may be mineral or organic, and may be in form of spherical particles, or lamellar particles.

In the present patent application, the term "spherical particles" means particles in the form or substantially in the form of a sphere, which are insoluble in the medium of the composition according to the invention, even at the melting point of the medium (about 100°C).

The term "lamellar particles" means herein particles of parallelepipedal shape (rectangular or square surface), discoid shape (circular surface) or ellipsoid shape (oval surface), characterized by three dimensions: a length, a width and a height, these particles being insoluble in the medium of the composition according to the invention, even at the melting point of the medium (about 100°C).

More particularly, the hydrophobic particles are chosen from:

### - hydrophobic silica,

The term "hydrophobic silica" is understood to mean, in the context of the present invention, both pure hydrophobic silicas and particles coated with hydrophobic silica.

According to a specific embodiment, the hydrophobic silicas which can be used in the composition of the invention are amorphous and of fumed origin. They are preferably provided in the pulverulent form.

The amorphous hydrophobic silicas of fumed origin are obtained from hydrophilic silicas. The latter are obtained by pyrolysis of silicon tetrachloride (SiCl₄) in a continuous flame at 1000°C in the presence of hydrogen and oxygen. They are subsequently rendered hydrophobic by treatment with halogenated silanes, alkoxysilanes or silazanes. The hydrophobic silicas differ from the starting hydrophilic silicas, inter alia, in a lower density of silanol groups and in a smaller adsorption of water vapour.

According to this embodiment, the hydrophobic silica is preferably chosen from silicas having a specific surface of from 50 to 500 m²/g and a number-average particle size ranging from 3 to 50 nm. These are more particularly the hydrophobic silicas described in the following table, and their mixtures.

| Commercial name | Aerosil R202 (Evonik Degussa) | Aerosil R805 (Evonik Degussa) | Aerosil R812 (Evonik Degussa) | Aerosil R972 (Evonik Degussa) | Aerosil R974 (Evonik Degussa) |
|---|---|---|---|---|---|
| BET surface (m2/g) | 90 + 20 | 150 + 25 | 260 + 30 | 110 + 20 | 170 + 20 |
| Average particle size (nm) | 14 | 12 | 7 | 1 | 12 |

According to this embodiment, the hydrophobic silica used in the composition of the invention can also consist of a particle completely or partially covered with silica, in particular of an inorganic particle completely or partially covered with hydrophobic silica, such as pigments and metal oxides covered with hydrophobic silica. These particles can also have optical properties in the product and on the skin; for example, they can have a mattifying or slightly whitening effect.

Use is preferably made, as hydrophobic silica, of a hydrophobic fumed silica treated at the surface with a dimethylsiloxane, such as that sold under the name Aerosil R972 (INCI name: Silica Dimethyl Silylate) by Evonik Degussa.

According to another specific embodiment, the hydrophobic silica which can be used in the composition of the invention are aerogel particles of hydrophobic silica exhibiting a specific surface per unit of weight (SW) ranging from 500 to 1500 m²/g and a size, expressed as volume-average diameter (D[0.5], also known as median particle size by volume Dv50), ranging from 1 to 1500 µm.

Silica aerogels are porous materials obtained by replacing (by drying) the liquid component of a silica gel with air.

They are generally synthesized via a sol-gel process in a liquid medium and then dried, usually by extraction of a supercritical fluid, the one most commonly used being supercritical CO₂. This type of drying makes it possible to avoid shrinkage of the pores and of the material. The sol-gel process and the various drying operations are described in detail in Brinker C.J. and Scherer G.W., Sol-Gel Science: New York: Academic Press, 1990.

The aerogel particles of hydrophobic silica used in the present invention exhibit a specific surface per unit of weight (SW) ranging from 500 to 1500 m²/g, preferably from 600 to 1200 m²/g and better still from 600 to 800 m²/g, and a size, expressed as volume-average diameter (D[0.5], also known as median particle size by volume Dv50), ranging from 1 to 1500 µm, better still from 1 to 1000 µm, preferably from 1 to 100 µm, in particular from 1 to 30 µm, more preferably from 5 to 25 µm, better still from 5 to 20 µm and even better still from 5 to 15 µm.

According to one embodiment, the aerogel particles of hydrophobic silica used in the present invention exhibit a size, expressed as volume-average diameter (D[0.5], also known as median particle size by volume Dv50), ranging from 1 to 30 µm, preferably from 5 to 25 µm, better still from 5 to 20 µm and even better still from 5 to 15 µm.

The specific surface per unit of weight can be determined by the nitrogen absorption method, known as the BET (Brunauer-Emmett-Teller) method, described in The Journal of the American Chemical Society, Vol. 60, page 309, February 1938, and corresponding to the international standard ISO 5794/1 (appendix D). The BET specific surface corresponds to the total specific surface of the particles under consideration. The sizes of the aerogel silica particles can be measured by static light scattering using a commercial particle size analyser of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., "Light Scattering by Small Particles", Chapters 9 and 10, Wiley, New York, 1957.

According to an advantageous embodiment, the aerogel particles of hydrophobic silica used in the present invention exhibit a specific surface per unit of weight (SW) ranging from 600 to 800 m²/g and a size, expressed as volume-average diameter (D[0.5], also known as median particle size by volume Dv50), ranging from 5 to 20 µm and even better still from 5 to 15 µm.

The aerogel silica particles used in the present invention can advantageously exhibit a packed density (p) ranging from 0.04 g/cm3 to 0.10 g/cm3 and preferably from 0.05 g/cm3 to 0.08 g/cm3.

In the context of the present invention, this density, known as the packed density, can be assessed according to the following protocol:
40 g of powder are poured into a graduated measuring cylinder; the measuring cylinder is then placed on the Stav 2003 device from Stampf Volumeter; the measuring cylinder is subsequently subjected to a series of 2500 packing actions (this operation is repeated until the difference in volume between two consecutive tests is less than 2%); the final volume Vf of packed powder is then measured directly on the measuring cylinder. The packed density is determined by the ratio w/Vf, in the case in point 40/Vf (Vf being expressed in cm3 and w in g).

According to one embodiment, the aerogel particles of hydrophobic silica used in the present invention exhibit a specific surface per unit of volume SV ranging from 5 to 60 m²/cm³, preferably from 10 to 50 m²/cm³ and better still from 15 to 40 m²/cm³.

The specific surface per unit of volume is given by the relationship: SV = SW x p where p is the packed density, expressed in g/cm³, and SW is the specific surface per unit of weight, expressed in m²/g, as defined above.

Preferably, the aerogel particles of hydrophobic silica according to the invention have an oil absorption capacity, measured at the wet point, ranging from 5 to 18 ml/g, preferably from 6 to 15 ml/g and better still from 8 to 12 ml/g.

The absorption capacity measured at the wet point, denoted Wp, corresponds to the amount of oil which it is necessary to add to 100 g of particles in order to obtain a homogeneous paste.

It is measured according to the "wet point" method or method for determining the oil uptake of a powder described in Standard NF T 30-022. It corresponds to the amount of oil adsorbed onto the available surface of the powder and/or absorbed by the powder by measurement of the wet point, described below:
An amount w = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm and smooth paste is obtained. This paste must be able to be spread on the glass plate without cracking or forming lumps. The volume Vs (expressed in ml) of oil used is then noted.

The oil uptake corresponds to the ratio Vs/w.

The aerogels used according to the present invention are hydrophobic silica aerogels, preferably of silylated silica (INCI name: silica silylate).

The term "hydrophobic silica" is understood to mean any silica whose surface is treated with silylating agents, for example with halogenated silanes, such as alkylchlorosilanes, siloxanes, in particular dimethylsiloxanes, such as hexamethyldisiloxane, or silazanes, so as to functionalize the OH groups with silyl groups Si-Rn, for example trimethylsilyl groups.

As regards the preparation of aerogel particles of hydrophobic silica modified at the surface by silylation, reference may be made to the document US 7 470 725.

Use will in particular be made of aerogel particles of hydrophobic silica modified at the surface with trimethylsilyl groups (trimethylsiloxylated silica).

Mention may be made, as hydrophobic silica aerogels which can be used in the invention, for example, of the aerogel sold under the name VM-2260 (INCI name: Silica silylate) by Dow Corning, the particles of which exhibit an average size of approximately 1000 microns and a specific surface per unit of weight ranging from 600 to 800 m²/g.

Mention may also be made of the aerogels sold by Cabot under the references Aerogel TLD 201, Aerogel OGD 201 and Aerogel TLD 203.

Use will more particularly be made of the aerogel sold under the name VM-2270 (INCI name: Silica silylate) by Dow Corning, the particles of which exhibit an average size ranging from 5 to 15 microns and a specific surface per unit of weight ranging from 600 to 800 m²/g.
- hydrophobic cellulose, for example alkyl cellulose; mentions may be made of the product ethyl cellulose sold under the trade name Ethocel™ Standard 200 Industrial Etnylcellulose from Dow Chemicals,
- starches,

All the starches and flours are suitable for use herein and may be derived from any native source. Preferably mention may be made of hydrophobic or hydrophobically modified starches. Also suitable are starches and flours derived from a plant obtained by standard breeding techniques including crossbreeding, translocation, inversion, transformation or any other method of gene or chromosome engineering to include variations thereof. In addition, starch or flours derived from a plant grown from artificial mutations and variations of the above generic composition which may be produced by known standard methods of mutation breeding are also suitable herein. Typical sources for the starches and flours are cereals, tubers, roots, legumes and fruits. The native source can be corn, pea, potato, sweet potato, banana, barley, wheat, rice, sago, amaranth, tapioca, arrowroot, canna, sorghum, and waxy or high amylose varieties thereof.

In particular, hydrophobically modified starches according to the present invention are preferred. Such starches include, for example, aluminum starch octenylsuccinate. Aluminum starch octenylsuccinate is commonly sold under the tradename DRY-FLO PURE by the company Akzo Nobel.

The starch may be first nonionically derivatized using an ester or ether which is compatible with the system, particularly with the solvent. Methods of nonionically derivatization are well known in the art and may be found for example in Starch Chemistry and Technology, 2nd ed., Edited by Whistler, et al., Academic Press, Inc., Orlando (1984) or Modified Starches: Properties and Uses. Wurzburg, O. B., CRC Press, Inc., Florida, (1986).

Nonionic reagents include, but are not limited to alkylene oxides such as ethylene oxide, propylene oxide, and butylene oxide, acetic anhydride, and butyl ketene dimer. Particularly suitable nonionic reagents are the alkylene oxides, more particularly propylene oxide.

Typically, the modified starches are powders at room temperature and atmospheric pressure. The modified starch powders are fine-grained. Further, the modified starch of the present invention has a particle size distribution of 5-30 microns and an average particle size of 15 microns. Moreover, the refractive index of the modified starch is measured to be between 1.50 and 1.60 at 25°C, preferably 1.52.

### - talc

The hydrophobic particle may be chosen from talc.

More particularly, the talc is micro-talc (for instance Micro Ace P3 by Nippon Talc.

Micro-talc particle sizes preferably range from 1 to 300 µm; most preferably ranging from 2 to 15 µm. The talc particles may be used alone or in combination. Hybrid powders may be employed, including talc in combination with titanium dioxide, aluminum oxide, and silicon (for instance Coverleaf AR80 from Presperse LLC), talc in combination with aluminum oxide and silicone dioxide (for instance Coverleaf AR100).

Other hybrid powder contemplated include zinc oxide on mica-barium sulfate (for instance Shadeleaf A from Merck), titanium dioxide on mica (for instance Blancsealer from Merck), titanium dioxide on silica (for instance NL T30H2WA from Nippon Sheet Glass), and titanium dioxide on mica-barium sulfate (for instance Naturaleaf powder from Merck).

Micro-talc is preferred in accordance to the present invention.

### - Silicone resin powders,

The preferred silicone resin powder is, for instance the silicone resin with the INCI name polymethylsilsesquioxane sold under the trade name Tospearl 145A by the company GE Silicone, with a mean size of 4.5 microns.
- hollow hemispherical silicone particles, for instance methylsilanol/silicate crosspolymer sold under the trade name NLK 500, NLK 506 and NLK 510 by the company Takemoto Oil and Fat,
- polyamide (Nylon®) powders, for instance Nylon 12 particles of the SP-500 from Toray Industries,
- hydrophobic pigments,

The hydrophobic pigments of the present invention may be hydrophobic or hydrophobic coated pigments. The hydrophobic coated pigments present in the emulsion according to the invention are pigments which are surface-treated with a hydrophobic agent. These treated pigments are well dispersed in the fatty phase.

As hydrophobic pigments, it is possible to use metal oxides such as iron oxides (in particular those which are yellow, red, brown or black in colour), titanium dioxides, cerium oxide, zirconium oxide, chromium oxide; manganese violet, ultramarine blue, Prussian blue, ferric blue, bismuth oxychloride, pearl, mica coated with titanium dioxyde or with bismuth oxychloride, coloured pearlescent pigments such as mica-titanium with iron oxides, mica-titanium with in particular ferric blue or chromium oxide, mica-titanium with an organic pigment of the abovementioned type and pearlescent pigments based on bismuth oxychloride, and mixtures thereof.

Hydrophobic pigments of iron oxides or of titanium dioxide are preferably used.

The hydrophobic treatment agent may be chosen from silicones such as methicones, dimethicones, perfluoroalkylsilanes; fatty acids such as stearic acid; metal soaps such as aluminium dimyristate, the aluminium salt of hydrogenated tallow glutamate, perfluoroalkyl phosphates, perfluoroalkylsilanes, perfluoroalkylsilazanes, polyhexafluoropropylene oxides, polyorganosiloxanes comprising perfluoroalkyl perfluoropolyether groups, amino acids; N-acylated amino acids or their salts; lecithin, isopropyl triisostearyl titanate, and mixtures thereof.

Preferably, fatty acids such as stearic acid is used as the hydrophobic treatment agent.

Mentions may be made of the hydrophobic coated pigment, such as metal oxides coated with fatty acids, for example titanium dioxide and aluminum hydroxide coated with stearic acid, which is sold under the tradename Micro Titanium Dioxide MT-100 T V by the company Tayca.

### - or a mixture thereof.

According to one embodiment, the composition of the present invention comprises at least onehydrophobic particle chosen from hydrophobic silica, starches, hydrophobic pigments, or a mixture thereof.

More preferably, the composition of the present invention comprises at least one hydrophobic particle chosen from hydrophobic fumed silica treated at the surface with a dimethylsiloxane, aerogel particles of hydrophobic silica exhibiting a specific surface per unit of weight (SW) ranging from 500 to 1500 m²/g and a size, expressed as volume-average diameter (D[0.5], also known as median particle size by volume Dv50), ranging from 1 to 1500 µm, hydrophobic cellulose such as ethyl cellulose, hydrophobically modified starches, hydrophobic pigments, or a mixture thereof.

Even more preferably, the composition of the present invention comprises at least one hydrophobic particle chosen from silica dimethyl silylate, silica silylate, alkyl cellulose such as ethyl cellulose, aluminum starch octenylsuccinate, pigments of iron oxides or of titanium dioxide, pigments of metal oxides coated with fatty acids such as stearic acid, or a mixture thereof.

The composition of the present invention comprises from 0.001% to 5% by weight, preferably from 0.05% to 2% by weight of the hydrophobic particles, relative to the total weight of the composition.

### Polysaccharide

The composition according to the invention comprises at least one polysaccharide of biotechnological origin.

In particular, these polysaccharides may, where appropriate, be chemically modified to promote its hydrophilic valency, as is the case for cellulose derivatives, in particular hydroxyalkylcelluloses (e.g.: hydroxyethylcellulose).

As examples of polysaccharides that may be used according to the invention, mention may be made especially of:
a) algal extracts such as alginates, carrageenans and agar-agar, and mixtures thereof. Examples of carrageenans that may be mentioned include Satiagum UTC30® and UTC10® from the company Degussa; an alginate that may be mentioned is the sodium alginate sold under the name Kelcosol® by the company ISP;
b) gums, such as xanthan gum, gellan gum, guar gum and nonionic derivatives thereof (hydroxypropyl guar), gum arabic, konjac gum or mannan gum, gum tragacanth, ghatti gum, karaya gum, locust bean gum, agar gum, scleroglucan gums and mixtures thereof; examples that may be mentioned include the xanthan gum sold under the name Keltrol® CG-T by the company CP Kelco, gellan gum sold under the name Kelcogel® CG LA by the company CP Kelco, guar gum sold under the name Jaguar HP 105® by the company Rhodia; mannan gum and konjac gum® (1% glucomannan) sold by the company GfN;
c) starches, which are preferably modified, such as those derived, for example, from cereals such as wheat, corn or rice, from legumes such as white lentil, from tubers such as potato or cassava, tapioca starches; dextrins, such as corn dextrins; Amidon de Maïs B® from the company Roquette; potato feculent modified with 2-chloroethylaminodipropionic acid neutralized with sodium hydroxide, sold under the name Structure Solanace® by the company National Starch; native tapioca starch powder sold under the name Tapioca Pure® by the company National Starch;
d) dextrins, such as dextrin extracted from corn under the name Index® from the company National Starch;
e) celluloses and derivatives thereof, in particular alkyl or hydroxyalkylcelluloses; mention may be made especially of methylcelluloses, hydroxyalkylcelluloses, ethylhydroxyethylcelluloses and carboxymethylcelluloses. Examples that may be mentioned include the hydroxyethylcellulose sold under the name Natrosol™ 250 HHR PC by the company Ashland, or under the name Cellosize™ QP 4400 H by the company Amerchol (Dow Chemical), cetylhydroxyethylcelluloses sold under the names Polysurf 67CS® and Natrosol Plus 330® from Aqualon;
f) pectins,
g) chitosan and derivatives thereof,
h) polyholosides comprising at least two saccharides, preferably of natural origin, and especially chosen from:
   - aldoses such as
      - pentoses: ribose, arabinose, xylose or apiose, for example,
      - hexoses: glucose, fucose, mannose or galactose, for example,
   - ketoses such as fructose,
   - deoxyoses, such as rhamnose, digitoxose, cymarose or oleandrose,
   - saccharide derivatives such as uronic acids, for instance mannuronic acid, guluronic acid, galacturonic acid or glycuronic acid, or itols, for instance mannitol or sorbitol.

Mention may be made in particular of the polyholosides comprising fucose, galactose and galacturonic acid units, and for example a linear sequence of α-L-fucose, α-D-galactose and galacturonic acid, for instance the biosaccharide gum-1 sold under the trade name Fucogel® 1000 PP or Fucogel® 1.5P by the company Solabia,
i) anionic polysaccharides, in particular of biotechnological origin, such as anionic polysaccharide bearing as repeating unit a tetrasaccharide composed of L-fucose, D-glucose and glucuronic acid, such as the product bearing the INCI name Biosaccharide Gum-4 sold under the reference Glycofilm 1.5P by the company Solabia,
j) and mixtures thereof.

Preferably, the polysaccharide of the present invention is chosen from:
- gums such as xanthan gum, or gellan gum;
- cellulose and its derivatives, such as hydroxyethylcellulose;
- polyholosides comprising fucose, galactose and galacturonic acid units, for example biosaccharide gum-1.

According to a preferred embodiment, the present invention comprises from 0.0001% to 5% by weight, preferably from 0.001% to 2% by weight, more preferably from 0.005% to 1% by weight of the polysaccharides, relative to the total weight of the composition.

### Additives

In a particular embodiment, a composition according to the invention further comprises at least one compound chosen from hydrophilic solvents, lipophilic solvents, additional oils, and mixtures thereof.

A cosmetic composition according to the invention may also comprise any additive usually used in the field under consideration, chosen, for example, from fillers or viscosity increasing agents, gelling agents, additional gums, additional resins, additional thickening agents, structuring agents such as waxes, dispersants, antioxidants, essential oils, preserving agents, fragrances, neutralizers, antiseptics, additional UV-screening agents, cosmetic active agents, such as vitamins, moisturizers, emollients or collagen-protecting agents, and mixtures thereof.

Suitable fillers and/or viscosity increasing agents include silicate clays such as, for example, silicate clays containing at least one cation which may be chosen from calcium, magnesium, aluminium, sodium, potassium, and lithium cations, and mixtures thereof. Non-limiting examples of such products include smectite clays such as montmorillonites, hectorites, bentonites, beidellites, saponites, vermiculites, stevensite, and chlorites. Preferred examples of silicate clays which may be used in the present invention are chosen from lithium magnesium silicate, aluminum calcium sodium silicate, calcium magnesium silicate, sodium magnesium silicate, calcium aluminum borosilicate, magnesium aluminum silicate, sodium potassium aluminum silicate, and sodium silver aluminum silicate.

If present, the fillers and/or viscosity increasing agents are present in an amount ranging from 0.05% to 10% by weight, preferably from 0.1% to 5% by weight, relative to the total weight of the composition.

Suitable gelling agents include esters of dextrin and fatty acids having a linear or branched, saturated or unsaturated C₁₂-C₂₄, in particular C₁₄-C₁₈ carbon chain. More particularly mentions may be made of these esters, for example dextrin myristate sold under the tradename Rheopearl MKL-2 by the company CHIBA Flour, or dextrin palmitate sold under the tradename Rheopearl TL2-OR by the company CHIBA Flour; N-acylamino acid amides, for example diamides resulting from the action of an N-acylamino acid with amines containing from 1 to 22 carbon atoms, such as those described in WO-93/23008 [15], for example N-laurylglutamic acid dibutylamide, such as the product sold or manufactured by the company Ajinomoto under the name GP-1, or N-2-ethylhexanoylglutamic acid dibutylamide sold or manufactured by the company Ajinomoto under the name EB-21, of INCI name: Dibutyl Ethylhexanoyl Glutamide. Such a compound is described in patent application JP2005-298635.

When present, the gelling agents are in the fatty phase of the present invention, and is in an amount ranging from 0.05% to 10% by weight, more particularly from 0.1% to 5% by weight, relative to the total weight of the fatty phase. It is a matter of routine operations for a person skilled in the art to adjust the nature and amount of the additives present in the compositions in accordance with the invention such that the desired cosmetic properties and stability properties thereof are not thereby affected.

### Galenic form

The composition according to the invention is in form of an oil-in-water Pickering emulsion.

More particularly, the composition of the present invention has a fatty phase in form of droplets, in particular visible oil droplets, with a median particle size by volume Dv50 from 0.1 mm to 10 mm, preferably from 0.5 mm to 5 mm.

The composition of the present invention may have the appearance of a cream, a gel, particularly a transparent gel, an ointment, a milk, a lotion, a serum, a paste.

Preferably, the viscosity of the gel according to the invention is superior or equal to 10UD (Mobile 3) by Rheomat at 25°C.

The viscosity is generally measured at 25°C with a viscosimeter RHEOMAT RM 180 with Mobile 3 adapted to the viscosity of the product to be tested (mobile is chosen for having a measure between 10 and 90 for UD Unit Deviation), the measure being made after 10mn rotating the mobile inside the composition, with a cisaillement from 200s-1. The UD values may then be converted in Poises (1 Poise= 0.1Pa.s) with a correspondence table.

More preferably, the composition contains a gelified aqueous phase.

### Method and use

The composition of the present invention can be used for a non-theraputic process, such as a cosmetic process or method, for making up/caring for the keratin materials, such as the skin, in particular the face and the lips, by being applied to the skin, especially the face and the lips.

The present invention also relates to a use of the composition according to the present invention, as it is or in cosmetic product for making up/caring for/cleansing/make up removing products for the skin, especially for the face and the lips.

The examples that follow are aimed at illustrating the compositions and processes according to this invention, but are not in any way a limitation of the scope of the invention.

### EXAMPLES

### Example 1: formulation example

The following formulas were prepared (inv fla stands for invention formula, and compa fla stands for comparative formula):

| **Phase** | **INCI name** | **% of ingredient by active ingredient (wt%)** | | | | | | | |
|---|---|---|---|---|---|---|---|---|---|
| | | Inv fla 1 | Inv fla 2 | Inv fla 3 | Inv fla 4 | Inv fla 5 | Inv fla 6 | Compa fla 1 | Compa fla 2 |
| A | Silica Dimethyl Silylate (AEROSIL R 972 from NIK DEGUSSA) | 0.05 | 0.05 | 0 | 0 | 0.01 | 0 | 0.05 | 0.05 |
| | Ethyl cellulose (ETHOCEL ™ STANDARD 200 INDUSTRIAL ETHYLCELLULOS E from DOW CHEMICAL) | 0 | 0 | 0 | 0 | 0 | 0.005 | 0 | 0 |
| | Silica Silylate (VM-2270 AEROGEL FINE PARTICLES from DOW CORNING) | 0 | 0 | 0.05 | 0 | 0 | 0 | 0 | 0 |
| | Aluminum Starch Octenylsuccinate (DRY-FLO PURE from AKZO NOBEL) | 0 | 0 | 0 | 1.5 | 0 | 0 | 0 | 0 |
| | Alcohol | 5.00 | 5.00 | 5.00 | 5.00 | 5.00 | 5.00 | 5.00 | 5.00 |
| B | Xanthan Gum (KELTROL® CG-T from CP KELCO) | 0.00 4 | 0 | 0 | 0 | 0.1 | 0.1 | 0 | 0 |
| | Gellan gum (KELCOGEL CG LA, CP KELCO) | 0 | 0 | 0 | 0 | 0.1 | 0.1 | 0 | 0 |
| | Hydroxyethylcellulo se (CELLOSIZE™ QP 4400 H from AMERCHOL (DOW CHEMICAL)) | 0 | 0.00 4 | 0.00 4 | 0.04 | 0 | 0 | 0 | 0 |
| | PEG-8 (POLYGLYKOL 400 from CLARIANT) | 0 | 0 | 0 | 0 | 0 | 0 | 0.004 | 0 |
| | Carbomer (CARBOPOL 980 POLYMER from LUBRIZOL) | 0 | 0 | 0 | 0 | 0 | 0 | 0 | 0.004 |
| | Water | QS 100 | QS 100 | QS 100 | QS 100 | QS 100 | QS 100 | QS 100 | QS 100 |
| C | Diphenylsiloxy Phenyl Trimethicone (KF-56A from SHIN ETSU) | 20.00 | 20.00 | 20.00 | 20.00 | 4 | 4 | 20.00 | 20.00 |
| | Trimethyl Pentaphenyl Trisiloxane (PH-1555 HRI COSMETIC FLUID from DOW CORNING) | 0 | 0 | 0 | 0 | 1 | 1 | 0 | 0 |
| | CAPRYLIC/CAPRI C TRIGLYCERIDE (MYRITOL 318, COGNIS) | 0 | 0 | 0 | 0 | 0 | 0 | 0 | 0 |
| D | Dextrin Myristate (RHEOPEARL MKL2 from CHIBA FLOUR MILLING) | 0 | 0 | 0 | 0 | 0 | 0 | 0 | 0 |
| E | Calcium Chloride (EMPROVE® PH EUR,BP,JP,USP,F CC, E509 from MERCK) | 0 | 0 | 0 | 0 | 0.5 | 0.5 | 0 | 0 |
| F | Fragrance | 0.05 | 0.05 | 0.05 | 0.05 | 0.05 | 0.05 | 0.05 | 0.05 |
| | Colorant | 0.0001 | 0.0001 | 0.0001 | 0.0001 | 0.0001 | 0.0001 | 0.0001 | 0.0001 |

Remarks: comparative formulas 1 and 2 contain polymers which are not polysaccharides.

The formulas listed above were prepared following the steps of:
the ingredients of phase A were mixed under 25°C, using the mixer VMI RAYNERI produced by TURBOTEST, for 5 minutes;
phase B is well mixed using VMI RAYNERI for 20 minutes to get a clear solution under 25°C (or at 80 °C if xanthan gum and gellan gum were present in the formula) for 10 minutes, the mixture was cooled to 25 °C if the mixing was conducted at 80 °C;
mixing the ingredients to form the phase C, and adding the phase C into the mixture obtained from the above steps while stiring using the mixer VMI RAYNERI under 25 °C for 10 minutes; In Inv fla 5, the mixture obtained from the above steps and the phase C were heated to 80 °C during mixing, and then the mixture was cooled down to 25 °C;
phase E was added into the mixture obtained from the steps above, stirring the mixture using the device VMI RAYNERI for 5 minutes;
phase F was added into the mixture obtained from the steps above, stirring the mixture using the device VMI RAYNERI for 5 minutes.

### Example 2: evaluation example

The stability of the invention and comparative formulas prepared in the Example 1 were evaluated.

The stability tests of the invention formulas and the comparative formulas at 40°C, 45°C, and 65°C for two months were conducted using Binder oven (USA), by leaving the invention and comparative formulas in the oven for 2 months.

The stability tests at 4°C stability for two months were conducted using Zhongke Meiling refrigerator (YC-260L, China), by leaving the invention and comparative formulas in the refrigerator for 2 months.

The light stability tests for 24 hours were conducted using ATLAC (AMETEK Measurement and Calibration Technologies).

Lastly, the freezing-thaw stability tests were conducted for 10 cycles using Binder over (USA). In each cycle, the temperature will be changed gradually from 20°C to -20°C in 24 hours.

The result of the stability of the invention and comparative formulas were listed below.

| Test | | Inv fla 1 | Inv fla 2 | Inv fla 3 | Inv fla 4 | Inv fla 5 | Inv fla 6 | Compa fla 1 | Compa fla 2 |
|---|---|---|---|---|---|---|---|---|---|
| Stability test at different temperatures in 2 months | 4°C | OK | OK | OK | OK | OK | OK | OK | Cannot form visible oil beads |
| | 40°C | OK | OK | OK | OK | OK | OK | Oil beads coalesce after one month | NA |
| | 45°C | OK | OK | OK | OK | OK | OK | Oil beads coalesce after one month | NA |
| | 65°C | OK | OK | OK | OK | OK | OK | Oil beads coalesce after one month | NA |
| Light stability test | | OK | OK | OK | OK | OK | OK | OK | NA |
| Freezing-shaw stability test | | OK | OK | OK | OK | OK | OK | Oil beads coalesce after 2 cycles | NA |

It is observed from the result above, that the invention formulas 1 to 6 have aesthetic natures with visible oil droplets, whereas the comparative formula 2 has no visible oil droplets. Besides, the invention formula 1 to 6, with lower amount of ethanol, possess much better stabilities, comparing to the comparative formulas 1 and 2 under varied environmental conditions.

## Claims

1. A composition in form of an oil-in-water pickering emulsion comprising:
a) a dispersed fatty phase comprising at least one oil chosen from silicone oils, hydrocarbonated oils, or a mixture thereof;
b) a continuous aqueous phase comprising at least one C₁-C₄ alcohol in an amount ranging from 1% to 10% by weight, relative to the total weight of the composition, the at least one C₁-C₄ alcohol comprising ethanol, ethylene glycol, C₁-C₄ dialkylene glycol or a mixture thereof; and
c) 0.001% to 5% by weight, relative to the total weight of the composition, of at least one hydrophobic particle chosen from hydrophobic silicas, hydrophobic cellulose, starch, talc, silicone resin powders, hollow hemispherical silicone particles, polyamide powders, hydrophobic pigments, or a mixture thereof; and
d) at least one polysaccharide.

2. The composition of claim 1, wherein the dispersed fatty phase is in form of a droplet with a median particle size by volume Dv50 from 0.1 mm to 10 mm, preferably from 0.5 mm to 5 mm.

3. The composition of claim 1 or 2, wherein the dispersed fatty phase is present in an amount ranging from 0.1% to 40% by weight, preferably from 1% to 30% by weight, and more preferably from 3% to 20% by weight, relative to the total weight of the composition.

4. The composition of any one of the preceding claims 1 to 3, wherein the silicone oil is chosen from non volatile silicone oils such as non volatile phenyl silicone oils, non volatile non phenyl silicone oils, volatile silicone oils, or a mixture thereof, preferably chosen from
(a) non volatile phenyl silicone oils of formula (I), with or without dimethicone part, 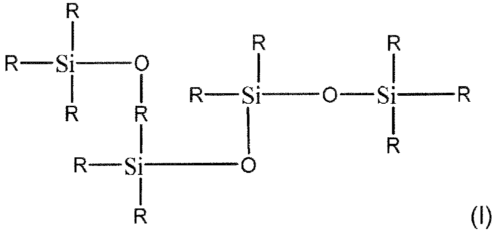 in which the groups R represent, monovalent or divalent, independently of each other, a methyl or a phenyl group, wherein at least 1, or 3, or 4, or 5, or even 6 group R represent phenyl group(s);
(b) non volatile phenyl silicone oils of formula (II), with or without dimethicone part, 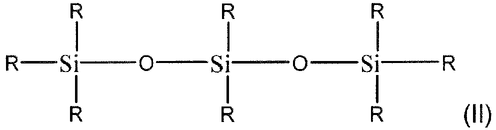 in which:
R represents, independently of each other, a methyl or a phenyl group, with the proviso that at least one, or three, or four, or five group R represent(s) phenyl group(s);
(c) non volatile phenyl silicone oils of formula (IV), with or without dimethicone part, 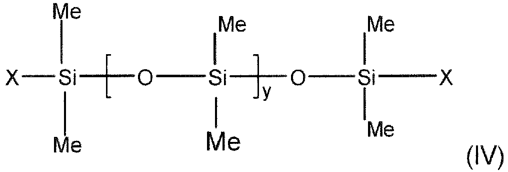 in which Me represents methyl, y is between 1 and 1,000 and X represents -CH₂-CH(CH₃)(Ph);
(d) non volatile phenyl silicone oils of formula (V), with or without dimethicone part, 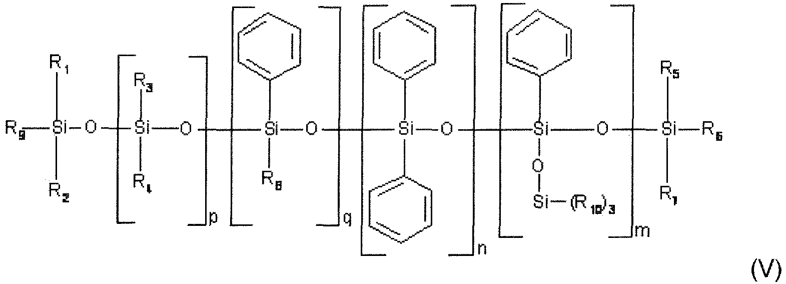 in which:
R₁ to R₁₀, independently of each other, are saturated or unsaturated, linear, cyclic or branched C₁-C₃₀ hydrocarbon-based radicals,
m, n, p and q are, independently of each other, integers between 0 and 900, with the proviso that the sum m+n+q is other than 0;
preferably, the sum m+n+q is between 1 and 100, more preferably, the sum m+n+p+q is between 1 and 900 and better still between 1 and 800, even more preferably, q is equal to 0, more preferably, R₁ to R₁₀, independently of each other, represent a saturated or unsaturated linear or branched C₁-C₃₀, preferably C₁-C₂₀, in particular C₁-C₁₆, hydrocarbon radical, preferably saturated, or a monocyclic or polycyclic C₆-C₁₄ and especially C₁₀-Cₗ₃ aryl radical, or an aralkyl radical, more preferably, R₁ to R₁₀ represent respecitively a methyl, ethyl, propyl, butyl, isopropyl, decyl, dodecyl or octadecyl radical, or alternatively a phenyl, tolyl, benzyl or phenethyl radical; R₁ to R₁₀ may especially be identical, and in addition may be a methyl radical;
(e) non volatile phenyl silicone oils of formula (IX), with or without dimethicone part, 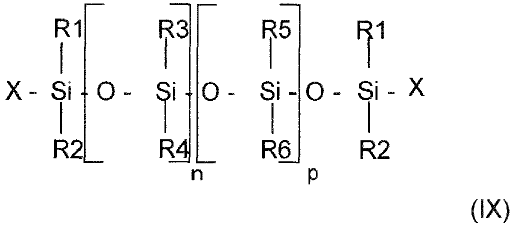 in which:
R₁, R₂, R₅ and R₆ are, together or separately, an alkyl radical containing 1 to 6 carbon atoms,
R₃ and R₄ are, together or separately, an alkyl radical containing from 1 to 6 carbon atoms or an aryl radical, with the proviso that at least one from R₃ and R₄ is a phenyl radical,
X is an alkyl radical containing from 1 to 6 carbon atoms, a hydroxyl radical or a vinyl radical,
n and p being integer superior or equal to 1;
(f) non volatile polydimethylsiloxanes;
(g) volatile polydimethylsiloxanes, linear or cyclic, having from 3 to 7 silicon atoms;
(h) or a mixture thereof.

5. The composition of any one of the preceding claims 1 to 4, wherein the silicone oil is chosen from phenyl trimethicones, diphenylsiloxyphenyltrimethicones, trimethylpentaphenyl trisiloxanes, trimethylsiloxyphenyldimethicones, or a mixture thereof.

6. The composition of any one of the preceding claims 1 to 5, wherein the hydrocarbonated oil is chosen from non volatile hydrocarbonated apolar oils, non volatile hydrocarbonated polar oils, volatile hydrocarbonated oils, or a mixture thereof; preferably the hydrocarbonated oil is chosen from hydrocarbonated plant oils, ester oils, fatty alcohols containing from 12 to 26 carbon atoms, fatty acids containing from 12 to 26 carbon atoms, dialkyl carbonates, vinylpyrrolidone copolymers, or a mixture thereof; more preferably, the hydrocarbonated oil is chosen from hydrocarbonated plant oils, more preferably heptanoic/octanoic triglycerides, caprylic/capric triglycerides, jojoba oils, or a mixture thereof.

7. The composition of any one of the preceding claims 1 to 6, wherein the oil is present in an amount ranging from 0.1% to 40% by weight, preferably from 1% to 30% by weight, and more preferably from 3% to 20% by weight, relative to the total weight of the composition.

8. The composition of any one of the claims 1 to 7, wherein the C₁-C₄ alcohol is ethanol.

9. The composition of any one of the preceding claims 1 to 8, wherein the aqueous phase is present in an amount ranging from 55% to 99.9% by weight, especially from 60% to 90% by weight, and more particularly from 65% to 85% by weight, relative to the total weight of the composition.

10. The composition of any one of the preceding claims 1 to 9, wherein the hydrophobic particle is chosen from silica dimethyl silylate, silica silylate, hydrophobic alkyl cellulose, aluminum starch octenylsuccinate, micro-talc, polymethylsilsesquioxane, methylsilanol/silicate crosspolymer, nylon-12, metal oxides, metal oxides coated with fatty acids, or a mixture thereof; more preferably, the hydrophobic particle is chosen from silica dimethyl silylate, silica silylate, ethyl cellulose, aluminum starch octenylsuccinate, titanium dioxide, iron oxides, or a mixture thereof.

11. The composition of any one of the preceding claims 1 to 10, wherein the hydrophobic particle is present in an amount ranging from 0.05% to 2% by weight, relative to the total weight of the composition.

12. The composition of any one of the preceding claims 1 to 11, wherein the polysaccharide is chosen from algal extracts, gums, starches, dextrins, celluloses and derivatives thereof, pectins, chitosan and derivatives thereof, polyholosides comprising at least two saccharides, anionic polysaccharides, in particular of biotechnological origin, and mixtures thereof; more preferably the polysaccharide is chosen from gums such as xanthan gum, or gellan gum; cellulose and its derivatives, such as hydroxyethylcellulose; polyholosides comprising fucose, galactose and galacturonic acid units, for example biosaccharide gum-1, or a mixture thereof.

13. The composition of any one of the preceding claims 1 to 12, wherein the polysaccharide is present in an amount ranging from 0.0001% to 5% by weight, preferably from 0.001% to 2% by weight, more preferably from 0.005% to 1% by weight, relative to the total weight of the composition.

14. The composition of any one of the preceding claims 1 to 13 comprises at least one additive chosen from hydrophilic solvents, lipophilic solvents, additional oils, fillers or viscosity increasing agents, gelling agents, additional gums, additional resins, additional thickening agents, structuring agents such as waxes, dispersants, antioxidants, essential oils, preserving agents, fragrances, neutralizers, antiseptics, additional UV-screening agents, cosmetic active agents, such as vitamins, moisturizers, emollients or collagen-protecting agents, and mixtures thereof; preferably chosen from fillers or viscosity increasing agents, gelling agents, or a mixture thereof; more preferably chosen from dextrin palmitate, dextrin myristate, N-laurylglutamic acid dibutylamide, N-2-ethylhexanoylglutamic acid dibutylamide, or a mixture thereof.

15. A process for making up/caring for the keratin materials, for example the skin, in particular the face and the lips, by applying to the keratin materials the composition according to any one of the previous claims 1 to 14.

## Patentansprüche

1. Zusammensetzung in Form einer ÖI-in-Wasser-Pickering-Emulsion, die Folgendes umfasst:
a) eine dispergierte Fettphase, die mindestens ein Öl umfasst, das aus Siliconölen, kohlenwasserstoffhaltigen Ölen oder einer Mischung davon ausgewählt ist;
b) eine kontinuierliche wässrige Phase, die mindestens einen C₁-C₄-Alkohol in einer Menge im Bereich von 1 bis 10 Gew.-%, bezogen auf das Gesamtgewicht der Zusammensetzung, umfasst, wobei der mindestens eine C₁-C₄-Alkohol Ethanol, Ethylenglycol, C₁-C₄-Dialkylenglycol oder eine Mischung davon umfasst; und
c) 0,001 bis 5 Gew.-%, bezogen auf das Gesamtgewicht der Zusammensetzung, mindestens eines hydrophoben Partikels, das aus hydrophoben Kieselsäuren, hydrophober Cellulose, Stärke, Talkum, Siliconharzpulvern, hohlen halbkugelförmigen Siliconpartikeln, Polyamidpulvern, hydrophoben Pigmenten oder einer Mischung davon ausgewählt ist; und
d) mindestens ein Polysaccharid.

2. Zusammensetzung nach Anspruch 1, wobei die dispergierte Fettphase in Form eines Tröpfchens mit einer volumengemittelten Partikelgröße Dv50 von 0,1 mm bis 10 mm, vorzugsweise von 0,5 mm bis 5 mm vorliegt.

3. Zusammensetzung nach Anspruch 1 oder 2, wobei die dispergierte Fettphase in einer Menge im Bereich von 0,1 bis 40 Gew.-%, vorzugsweise von 1 bis 30 Gew.-% und mehr bevorzugt von 3 bis 20 Gew.-% vorliegt, bezogen auf das Gesamtgewicht der Zusammensetzung.

4. Zusammensetzung nach einem der Ansprüche 1 bis 3, wobei das Siliconöl aus nicht flüchtigen Siliconölen wie zum Beispiel nicht flüchtigen Phenylsiliconölen, nicht flüchtigen phenylfreien Siliconölen, flüchtigen Siliconölen oder einer Mischung davon ausgewählt ist, vorzugsweise ausgewählt ist aus
(a) nicht flüchtigen Phenylsiliconölen der Formel (I), mit oder ohne Dimethiconteil, 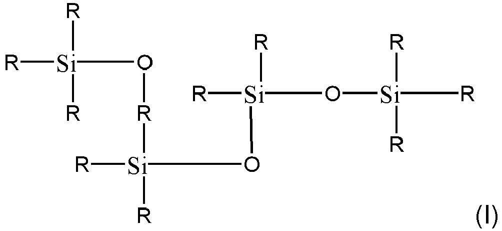 bei der die R-Gruppen, einwertig oder zweiwertig, unabhängig voneinander eine Methylgruppe oder eine Phenylgruppe darstellen, wobei mindestens 1 oder 3 oder 4 oder 5 oder sogar 6 R-Gruppen Phenylgruppe(n) darstellt/darstellen;
(b) nicht flüchtige Phenylsiliconöle der Formel (II), mit oder ohne Dimethiconteil, 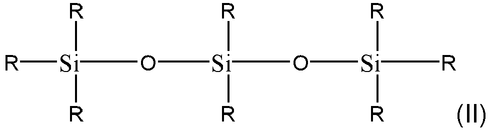 bei der:
R, unabhängig voneinander, eine Methylgruppe oder eine Phenylgruppe darstellt, mit der Maßgabe, dass mindestens eine oder drei oder vier oder fünf R-Gruppen eine oder mehrere Phenylgruppen darstellt/darstellen;
(c) nicht flüchtige Phenylsiliconöle der Formel (IV), mit oder ohne Dimethiconteil, 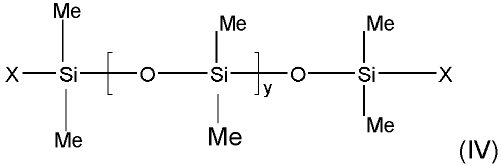 bei der Me für Methyl steht, y zwischen 1 und 1000 liegt und X für -CH₂-CH(CH₃)(Ph) steht;
(d) nicht flüchtige Phenylsiliconöle der Formel (V), mit oder ohne Dimethiconteil, 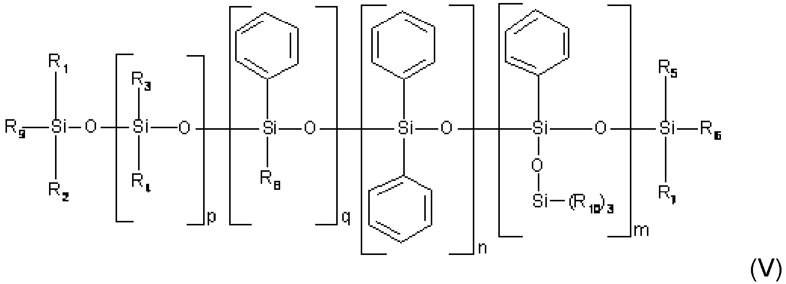 bei der:
R₁ bis R₁₀, unabhängig voneinander, gesättigte oder ungesättigte, lineare, cyclische oder verzweigte C₁-C₃₀-Radikale auf Kohlenwasserstoffbasis sind, m, n, p und q, unabhängig voneinander, ganze Zahlen zwischen 0 und 900 sind, mit der Maßgabe, dass die Summe m+n+q nicht 0 ist;
vorzugsweise beträgt die Summe m+n+q zwischen 1 und 100, mehr bevorzugt beträgt die Summe m+n+p+q zwischen 1 und 900 und besser noch zwischen 1 und 800, noch mehr bevorzugt ist q gleich 0; mehr bevorzugt repräsentieren R₁ bis R₁₀ unabhängig voneinander einen gesättigten oder ungesättigten linearen oder verzweigten Kohlenwasserstoffrest mit 1 bis 30, vorzugsweise 1 bis 20, insbesondere 1 bis 16 Kohlenstoffatomen, vorzugsweise einen gesättigten bzw. einen monocyclischen oder polycyclischen Arylrest mit 6 bis 14 und insbesondere 10 bis 13 Kohlenstoffatomen, oder einen Aralkylrest; mehr bevorzugt repräsentieren R₁ bis R₁₀ jeweils einen Methyl-, Ethyl-, Propyl-, Butyl-, Isopropyl-, Decyl-, Dodecyl- oder Octadecylrest oder alternativ einen Phenyl-, Tolyl-, Benzyl- oder Phenethylrest; R₁ bis R₁₀ können insbesondere identisch sein und können darüberhinaus ein Methylrest sein;
(e) nicht flüchtige Phenylsiliconöle der Formel (IX), mit oder ohne Dimethiconteil, 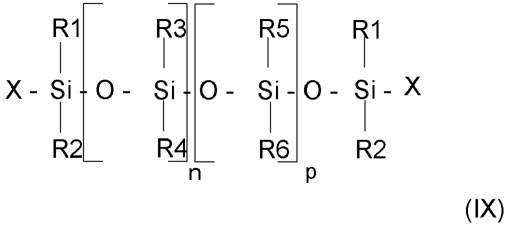 bei der:
R₁, R₂, R₅ und R₆, zusammen oder getrennt, einen Alkylrest darstellen, der 1 bis 6 Kohlenstoffatome enthält,
R₃ und R₄, zusammen oder getrennt, ein Alkylrest sind, der von 1 bis 6 Kohlenstoffatome enthält, oder ein Arylrest, mit der Maßgabe, dass mindestens eines von R₃ und R₄ ein Phenylrest ist,
X ein Alkylrest ist, der von 1 bis 6 Kohlenstoffatome enthält, ein Hydroxylrest oder ein Vinylrest,
wobei n und p ganze Zahlen größer oder gleich 1 sind;
(f) nicht flüchtige Polydimethylsiloxane;
(g) flüchtige Polydimethylsiloxane, linear oder cyclisch, mit 3 bis 7 Siliciumatomen;
(h) oder eine Mischung davon.

5. Zusammensetzung nach einem der vorhergehenden Ansprüche 1 bis 4, wobei das Siliconöl aus Phenyltrimethiconen, Diphenylsiloxyphenyltrimethiconen, Trimethylpentaphenyltrisiloxanen, Trimethylsiloxyphenyldimethiconen oder einer Mischung davon ausgewählt ist.

6. Zusammensetzung nach einem der vorhergehenden Ansprüche 1 bis 5, wobei das kohlenwasserstoffhaltige Öl aus nicht flüchtigen kohlenwasserstoffhaltigen apolaren Ölen, nicht flüchtigen kohlenwasserstoffhaltigen polaren Ölen, flüchtigen kohlenwasserstoffhaltigen Ölen oder einer Mischung davon ausgewählt ist; vorzugsweise ist das kohlenwassertoffhaltige Öl aus kohlenwasserstoffhaltigen Pflanzenölen, Esterölen, Fettalkoholen mit 12 bis 26 Kohlenstoffatomen, Fettsäuren mit 12 bis 26 Kohlenstoffatomen, Dialkylcarbonaten, Vinylpyrrolidoncopolymeren oder einer Mischung davon ausgewählt; mehr bevorzugt ist das kohlenwasserstoffhaltige Öl aus kohlenwasserstoffhaltigen Pflanzenölen ausgewählt, mehr bevorzugt aus Heptansäure/Octansäure-Triglyceriden, Caprylsäure/Caprinsäure-Triglyceriden, Jojobaölen oder einer Mischung davon.

7. Zusammensetzung nach einem der vorhergehenden Ansprüche 1 bis 6, wobei das Öl in einer Menge im Bereich von 0,1 bis 40 Gew.-%, vorzugsweise von 1 bis 30 Gew.-% und mehr bevorzugt von 3 bis 20 Gew.-% vorliegt, bezogen auf das Gesamtgewicht der Zusammensetzung.

8. Zusammensetzung nach einem der Ansprüche 1 bis 7, wobei es sich bei dem C₁-C₄-Alkohol um Ethanol handelt.

9. Zusammensetzung nach einem der vorhergehenden Ansprüche 1 bis 8, wobei die wässrige Phase in einer Menge im Bereich von 55 bis 99,9 Gew.-%, speziell von 60 bis 90 Gew.-% und insbesondere von 65 bis 85 Gew.-% vorliegt, bezogen auf das Gesamtgewicht der Zusammensetzung.

10. Zusammensetzung nach einem der vorhergehenden Ansprüche 1 bis 9, wobei das hydrophobe Partikel aus Kieselsäuredimethylsilylat, Kieselsäuresilylat, hydrophober Alkylcellulose, Aluminium-Stärke-Octenylsuccinat, Mikrotalkum, Polymethylsilsesquioxan, Methylsilanol/Silicat-Kreuzpolymer, Nylon-12, Metalloxiden, mit Fettsäuren beschichteten Metalloxiden oder einer Mischung davon ausgewählt ist; mehr bevorzugt ist das hydrophobe Partikel aus Kieselsäuredimethylsilylat, Kieselsäuresilylat, Ethylcellulose, Aluminium-Stärke-Octenylsuccinat, Titandioxid, Eisenoxiden oder einer Mischung davon ausgewählt.

11. Zusammensetzung nach einem der vorhergehenden Ansprüche 1 bis 10, wobei das hydrophobe Partikel in einer Menge im Bereich von 0,05 bis 2 Gew.-% vorliegt, bezogen auf das Gesamtgewicht der Zusammensetzung.

12. Zusammensetzung nach einem der vorhergehenden Ansprüche 1 bis 11, wobei das Polysaccharid aus Algenextrakten, Gummis, Stärken, Dextrinen, Cellulosen und Derivaten davon, Pektinen, Chitosan und Derivaten davon, Polyholosiden mit mindestens zwei Sacchariden, anionischen Polysacchariden, insbesondere biotechnologischen Ursprungs, und Mischungen davon ausgewählt ist; mehr bevorzugt ist das Polysaccharid aus Gummis wie zum Beispiel Xanthangummi oder Gellangummi ausgewählt; aus Cellulose und ihren Derivaten, wie zum Beispiel Hydroxyethylcellulose; aus Polyholosiden mit Fucose-, Galactose- und Galacturonsäureeinheiten, zum Beispiel Biosaccharidgummi-1, oder einer Mischung davon.

13. Zusammensetzung nach einem der vorhergehenden Ansprüche 1 bis 12, wobei das Polysaccharid in einer Menge im Bereich von 0,0001 bis 5 Gew.-%, vorzugsweise von 0,001 bis 2 Gew.-%, mehr bevorzugt von 0,005 bis 1 Gew.-% vorliegt, bezogen auf das Gesamtgewicht der Zusammensetzung.

14. Zusammensetzung nach einem der vorhergehenden Ansprüche 1 bis 13, mit mindestens einem Zusatzstoff, der aus hydrophilen Lösungsmitteln, lipophilen Lösungsmitteln, zusätzlichen Ölen, Füllstoffen oder die Viskosität erhöhenden Mitteln, Geliermitteln, zusätzlichen Gummis, zusätzlichen Harzen, zusätzlichen Verdickungsmitteln, Strukturmitteln wie zum Beispiel Wachsen, Dispergiermitteln, Antioxidationsmitteln, essentiellen Ölen, Konservierungsmitteln, Duftstoffen, Neutralisationsmitteln, Antiseptika, zusätzlichen UV-Filtern, kosmetischen Wirkstoffen wie zum Beispiel Vitaminen, Moisturizern, Emollients oder Kollagen schützenden Mitteln und Mischungen davon ausgewählt ist; vorzugsweise aus Füllstoffen oder die Viskosität erhöhenden Mitteln, Geliermitteln oder einer Mischung davon ausgewählt ist; mehr bevorzugt aus Dextrinpalmitat, Dextrinmyristat, N-Laurylglutaminsäuredibutylamid, N-2-Ethylhexanoylglutaminsäuredibutylamid oder einer Mischung davon.

15. Verfahren zum Schminken/Pflegen von Keratinmaterialien, zum Beispiel der Haut, insbesondere des Gesichts und der Lippen, durch Aufbringen der Zusammensetzung nach einem der vorhergehenden Ansprüche 1 bis 14 auf die Keratinmaterialien.

## Revendications

1. Composition sous la forme d'une émulsion d'huile dans l'eau de Pickering comprenant :
a) une phase grasse dispersée comprenant au moins une huile choisie parmi les huiles de silicone, les huiles hydrocarbonées, ou un mélange de celles-ci ;
b) une phase aqueuse continue comprenant au moins un alcool en C₁ à C₄ en une quantité allant de 1 % à 10 % en poids, par rapport au poids total de la composition, le au moins un alcool en C₁ à C₄ comprenant l'éthanol, l'éthylène glycol, un dialkylène glycol en C₁ à C₄ ou un mélange de ceux-ci ; et
c) 0,001 % à 5 % en poids, par rapport au poids total de la composition, d'au moins une particule hydrophobe choisie parmi les silices hydrophobes, une cellulose hydrophobe, un amidon, le talc, les poudres de résine de silicone, les particules de silicone hémisphériques creuses, les poudres de polyamide, les pigments hydrophobes, ou un mélange de ceux-ci ; et
d) au moins un polysaccharide.

2. Composition selon la revendication 1, dans laquelle la phase grasse dispersée est sous la forme d'une gouttelette ayant une taille particulaire médiane en volume Dv50 de 0,1 mm à 10 mm, de préférence de 0,5 mm à 5 mm.

3. Composition selon la revendication 1 ou 2, dans laquelle la phase grasse dispersée est présente en une quantité allant de 0,1 % à 40 % en poids, de préférence de 1 % à 30 % en poids, et de manière davantage préférée de 3 % à 20 % en poids, par rapport au poids total de la composition.

4. Composition selon l'une quelconque des revendications 1 à 3 précédentes, dans laquelle l'huile de silicone est choisie parmi les huiles de silicone non volatiles telles que les huiles de silicone phénylées non volatiles, les huiles de silicone non phénylées non volatiles, les huiles de silicone volatiles, ou un mélange de celles-ci, de préférence choisies parmi
(a) les huiles de silicone phénylées non volatiles de formule (I), avec ou sans partie diméthicone, 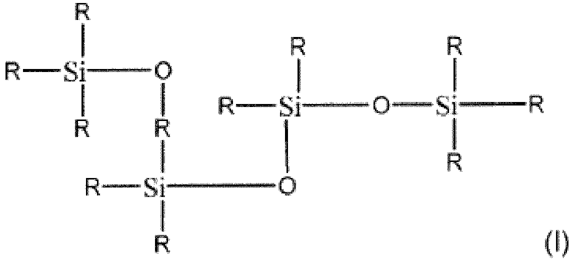 dans laquelle les groupes R représentent, monovalents ou divalents, indépendamment les uns des autres, un groupe méthyle ou phényle, dans laquelle au moins 1, ou 3, ou 4, ou 5, ou même 6 groupes R représentent un ou des groupes phényle ;
(b) les huiles de silicone phénylées non volatiles de formule (II), avec ou sans partie diméthicone, 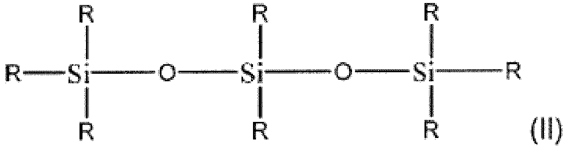 dans laquelle :
R représente, indépendamment les uns des autres, un groupe méthyle ou phényle, à condition qu'au moins un, ou trois, ou quatre, ou cinq groupes R représente (nt) un ou des groupe(s) phényle ;
(c) les huiles de silicone phénylées non volatiles de formule (IV), avec ou sans partie diméthicone, 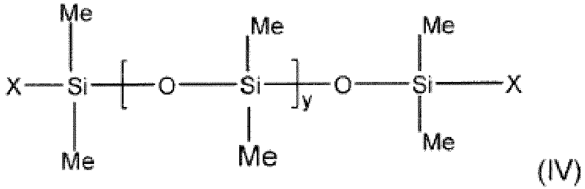 dans laquelle Me représente un méthyle, y est entre 1 et 1000 et X représente -CH₂-CH(CH₃) (Ph) ;
(d) les huiles de silicone phénylées non volatiles de formule (V), avec ou sans partie diméthicone, 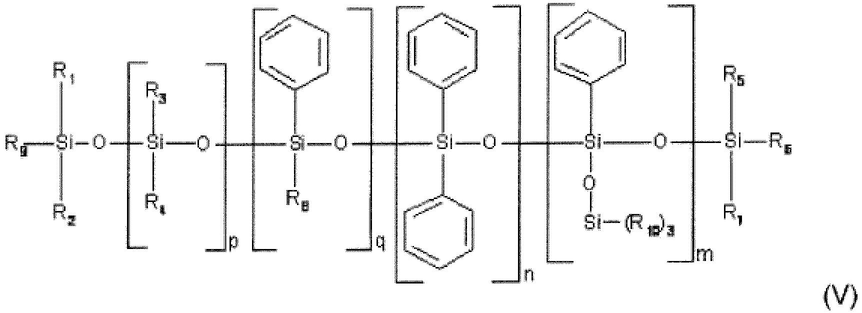 dans laquelle :
R₁ à R₁₀, indépendamment les uns des autres, sont des radicaux hydrocarbonés en C₁ à C₃₀, linéaires, cycliques ou ramifiés, saturés ou insaturés,
m, n, p et q sont, indépendamment les uns des autres, des nombres entiers entre 0 et 900, à condition que la somme m+n+q soit différente de 0 ;
de préférence, la somme m+n+q est entre 1 et 100, de manière davantage préférée, la somme m+n+p+q est entre 1 et 900 et encore mieux entre 1 et 800, de manière encore plus préférée, q est égal à 0, de manière davantage préférée, R₁ à R₁₀, indépendamment les uns des autres, représentent un radical hydrocarboné linéaire ou ramifié, saturé ou insaturé, en C₁ à C₃₀, de préférence en C₁ à C₂₀, en particulier en C₁ à C₁₆, de préférence saturé, ou un radical aryle monocyclique ou polycyclique en C₆ à C₁₄ et en particulier en C₁₀ à C₁₃, ou un radical aralkyle, de manière davantage préférée R₁ à R₁₀ représentent respectivement un radical méthyle, éthyle, propyle, butyle, isopropyle, décyle, dodécyle ou octadécyle, ou en variante un radical phényle, tolyle, benzyle ou phénéthyle ; R₁ à R₁₀ peuvent en particulier être identiques, et en plus peuvent être un radical méthyle ;
(e) les huiles de silicone phénylées non volatiles de formule (IX), avec ou sans partie diméthicone, 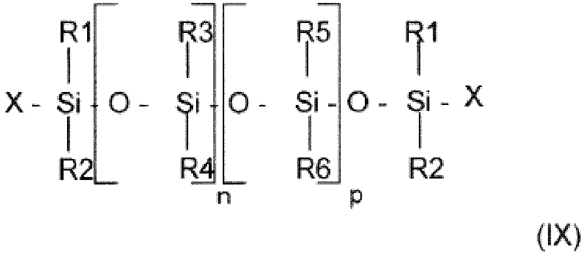 dans laquelle :
R₁, R₂, R₅ et R₆ sont, ensemble ou séparément, un radical alkyle contenant de 1 à 6 atomes de carbone,
R₃ et R₄ sont, ensemble ou séparément, un radical alkyle contenant de 1 à 6 atomes de carbone ou un radical aryle, à condition qu'au moins l'un parmi R₃ et R₄ soit un radical phényle,
X est un radical alkyle contenant de 1 à 6 atomes de carbone, un radical hydroxyle ou un radical vinyle,
n et p étant un nombre entier supérieur ou égal à 1 ;
(f) les polydiméthylsiloxanes non volatils ;
(g) les polydiméthylsiloxanes volatils, linéaires ou cycliques, ayant de 3 à 7 atomes de silicium ;
(h) ou un mélange de ceux-ci.

5. Composition selon l'une quelconque des revendications 1 à 4 précédentes, dans laquelle l'huile de silicone est choisie parmi les phényltriméthicones, les diphénylsiloxyphényltriméthicones, les triméthylpentaphényltrisiloxanes, les triméthylsiloxyphényldiméthicones, ou un mélange de celles-ci.

6. Composition selon l'une quelconque des revendications 1 à 5 précédentes, dans laquelle l'huile hydrocarbonée est choisie parmi les huiles hydrocarbonées apolaires non volatiles, les huiles hydrocarbonées polaires non volatiles, les huiles hydrocarbonées volatiles, ou un mélange de celles-ci ; de préférence l'huile hydrocarbonée est choisie parmi les huiles végétales hydrocarbonées, les huiles d'ester, les alcools gras contenant de 12 à 26 atomes de carbone, les acides gras contenant de 12 à 26 atomes de carbone, les carbonates de dialkyle, les copolymères de vinylpyrrolidone, ou un mélange de ceux-ci ; de manière davantage préférée, l'huile hydrocarbonée est choisie parmi les huiles végétales hydrocarbonées, de manière davantage préférée les triglycérides heptanoïques/octanoïques, les triglycérides capryliques/capriques, les huiles de jojoba, ou un mélange de ceux-ci.

7. Composition selon l'une quelconque des revendications 1 à 6 précédentes, dans laquelle l'huile est présente en une quantité allant de 0,1 % à 40 % en poids, de préférence de 1 % à 30 % en poids, et de manière davantage préférée de 3 % à 20 % en poids, par rapport au poids total de la composition.

8. Composition selon l'une quelconque des revendications 1 à 7, dans laquelle l'alcool en C₁ à C₄ est l'éthanol.

9. Composition selon l'une quelconque des revendications 1 à 8 précédentes, dans laquelle la phase aqueuse est présente en une quantité allant de 55 % à 99,9 % en poids, en particulier de 60 % à 90 % en poids, et de manière davantage préférée de 65 % à 85 % en poids, par rapport au poids total de la composition.

10. Composition selon l'une quelconque des revendications 1 à 9 précédentes, dans laquelle la particule hydrophobe est choisie parmi un diméthyl silylate de silice, un silylate de silice, une alkylcellulose hydrophobe, un octénylsuccinate d'aluminium et d'amidon, un microtalc, un polyméthylsilsesquioxane, un polymère réticulé de méthylsilanol/silicate, le nylon-12, les oxydes métalliques, les oxydes métalliques revêtus d'acides gras, ou un mélange de ceux-ci ; de manière davantage préférée, la particule hydrophobe est choisie parmi un diméthyl silylate de silice, un silylate de silice, une éthylcellulose, un octénylsuccinate d'aluminium et d'amidon, le dioxyde de titane, les oxydes de fer, ou un mélange de ceux-ci.

11. Composition selon l'une quelconque des revendications 1 à 10 précédentes, dans laquelle la particule hydrophobe est présente en une quantité allant de 0,05 % à 2 % en poids, par rapport au poids total de la composition.

12. Composition selon l'une quelconque des revendications 1 à 11 précédentes, dans laquelle le polysaccharide est choisi parmi les extraits d'algue, les gommes, les amidons, les dextrines, les celluloses et les dérivés de celles-ci, les pectines, le chitosane et les dérivés de celui-ci, les polyholosides comprenant au moins deux saccharides, les polysaccharides anioniques, en particulier d'origine biotechnologique, et les mélanges de ceux-ci ; de manière davantage préférée le polysaccharide est choisi parmi les gommes telles que la gomme xanthique, ou la gomme gellane ; la cellulose et ses dérivés, tels que l'hydroxyéthylcellulose ; les polyholosides comprenant des motifs fucose, galactose et acide galacturonique, par exemple la biosaccharide gomme-1, ou un mélange de ceux-ci.

13. Composition selon l'une quelconque des revendications 1 à 12 précédentes, dans laquelle le polysaccharide est présent en une quantité allant de 0,0001 % à 5 % en poids, de préférence de 0,001 % à 2 % en poids, de manière davantage préférée de 0,005 % à 1 % en poids, par rapport au poids total de la composition.

14. Composition selon l'une quelconque des revendications 1 à 13 précédentes comprenant au moins un additif choisi parmi les solvants hydrophiles, les solvants lipophiles, des huiles supplémentaires, les charges ou les agents augmentant la viscosité, les agents gélifiants, des gommes supplémentaires, des résines supplémentaires, des agents épaississants supplémentaires, les agents structurants tels comme les cires, les dispersants, les antioxydants, les huiles essentielles, les agents conservateurs, les parfums, les neutralisants, les antiseptiques, des agents filtrant les UV supplémentaires, les agents cosmétiques actifs, tels que les vitamines, les hydratants, les émollients ou les agents protégeant le collagène, et les mélanges de ceux-ci ; de préférence choisi parmi les charges ou les agents augmentant la viscosité, les agents gélifiants, ou un mélange de ceux-ci ; de manière davantage préférée choisi parmi le palmitate de dextrine, le myristate de dextrine, le dibutylamide de l'acide N-laurylglutamique, le dibutylamide de l'acide N-2-éthylhexanoylglutamique, ou un mélange de ceux-ci.

15. Procédé de maquillage/soin des matières kératiniques, par exemple la peau, en particulier le visage et les lèvres, par application sur les matières kératiniques de la composition selon l'une quelconque des revendications 1 à 14 précédentes.
